# EUROPÄISCHE PATENTSCHRIFT

(11) **EP 0 525 525 B1**
(45) Veröffentlichungstag und Bekanntmachung des Hinweises auf die Patenterteilung: **03.05.1995**
(21) Anmeldenummer: 92112138.0
(22) Anmeldetag: 16.07.1992
(51) Int. Cl.: A61M 5/20

(54) **Injektor**
Injector
Injecteur

(30) Priorität: 24.07.1991 DE 4124536
(43) Veröffentlichungstag der Anmeldung: 03.02.1993
(73) Patentinhaber: MEDICO DEVELOPMENT INVESTMENT COMPANY, CH-6612 Ascona (CH)
(72) Erfinder: Gabriel, Jochen, W-7000 Stuttgart 1 (DE); Bechtold, Herbert, W-7044 Ehningen (DE)
(74) Vertreter: Raible, Hans, Dipl.-Ing.

(56) Entgegenhaltungen:
- EP-A- 0 268 191
- WO-A-88/08724

## Beschreibung

Die Erfindung betrifft einen Injektor nach dem Oberbegriff des Patentanspruchs 1 oder des Patentanspruchs 23.

Ein derartiger Injektor ist bekannt aus der EP-A2-0 268 191, oder der EP-A-0 349 592. Diese bekannten Injektoren ermöglichen eine vollautomatische Injektion einer voreingestellten Dosis aus einer Kartusche, deren Inhalt gewöhnlich für eine Mehrzahl von Injektionen ausreicht. Die Bedienung ist hierdurch vereinfacht, erfordert aber doch von Seiten des Benutzers ein nicht unerhebliches Mitdenken, um Fehler sicher zu vermeiden, und man muß deshalb die Benutzer intensiv über die richtige Benutzung unterrichten.

Ferner kennt man aus der EP-A-0 338 806 einen Injektor zur vollautomatischen Injektion einer Flüssigkeit aus einem Vorratsbehälter. Dieses bekannte Gerät hat eine Feder für das Einstechen der Nadel, und eine zweite Feder für das automatische Injizieren der Flüssigkeit. Ist das Einstechen der Nadel beendet, so wird hierdurch die zweite Feder freigegeben, und dies bewirkt das Injizieren.

Das Injizieren erfolgt über eine Gewindespindel, die in einem Gehäuse längsverschiebbar, aber nicht verdrehbar, geführt ist. Zum Antrieb der Gewindespindel dient eine Freilaufkupplung, die zuvor auf eine gewünschte Dosis eingestellt wird und die nach dem Einstechen der Nadel durch ein Teil freigegeben wird, das bis dahin die Feilaufkupplung blockiert hatte. Die Feder treibt dann über die Freilaufkupplung ein Teil an, dessen Innengewinde die Gewindespindel axial verschiebt, und bewirkt so die Injektion. Durch die Vielzahl seiner Teile ist dieses Gerät kompliziert, und teuer in der Herstellung.

Es ist deshalb eine Aufgabe der Erfindung, einen neuen Injektor bereitzustellen.

Nach der Erfindung wird diese Aufgabe gelöst durch einen Injektor zur Aufnahme einer Kartusche mit einer gewöhlich für eine Mehrzahl von Injektionen ausreichenden Menge an Injektionsflüssigkeit, welche Kartusche im Injektor gegen die Kraft einer Rückstellfeder in proximaler Richtung verschiebbar ist,
mit einem durch eine Feder in proximaler Richtung beaufschlagten, längenverstellbaren Stößel, welcher im Injektor zwischen einer proximalen Endstellung und einer distalen Endstellung verschiebbar ist und eine Gewindespindel aufweist, die im Gewinde eines Verstellglieds geführt ist, zur Beaufschlagung eines in der Kartusche vorgesehenen Kolbens dient, und der ein Führungsglied zugeordnet ist, das mit ihr drehfest, aber axial verschiebbar, verbunden ist, wobei das Führungsglied in der distalen Endstellung des Stößels relativ zum Gehäuse des Injektors verdrehbar ist, in der proximalen Endstellung aber nicht. Man erreicht so, daß der eigentliche Injektionsvorgang, also das Injizieren der Flüssigkeit, erst ablaufen kann, wenn die Nadel bereits eingestochen hat, und daß sich nach der Injektion der Injektor in einer Stellung befindet, die vom Benutzer keine Rückstellvorgänge erfordert. Dadurch ergibt sich eine einfache, leicht verständliche Funktion.

Die Verdrehung des Verstellglieds relativ zum Führungsglied in der proximalen Endstellung des Stößels - zum Zwecke der Injektion von Injektionsflüssigkeit - kann von Hand erfolgen, doch geht man bevorzugt so vor, daß zwischen dem Gehäuse und dem Verstellglied eine Feder vorgesehen ist, welche durch Verdrehen des Verstellgliedes spannbar ist, und daß in der distalen Endstellung des Stößels eine Sperrvorrichtung vorgesehen ist, welche ein solches Verdrehen des Verstellgliedes nur in einer bestimmten Richtung ermöglicht und eine Drehung in der umgekehrten Richtung sperrt. Diese Feder kann also in der distalen Endstellung gespannt werden und sich dann in der proximalen Endstellung entspannen, um das Verstellglied relativ zum Führungsglied zu verdrehen und die Injektion der Injektionsflüssigkeit zu bewirken. Man geht dabei mit besonderem Vorteil so vor, daß die Sperrvorrichtung in der proximalen Endstellung des Stößels nicht wirksam ist.

Eine besonders vorteilhafte Weiterbildung der Erfindung ist dadurch gekennzeichnet, daß das Verstellglied in der distalen Endstellung es Stößels ausgehend von einer vorgegebenen Drehstellung durch Verdrehen verstellbar ist, und daß die Verdrehung des Verstellglieds in der proximalen Endstellung, die dort in umgekehrter Richtung abläuft, bis zu dieser vorgegebenen Drehstellung begrenzt ist. So erreicht man, daß sich das Gerät nach dem Injektionsvorgang automatisch wieder in einer Nullstellung befindet, so daß kein Rückstellvorgang erforderlich ist, um z.B. die Skala für die Injektionsdosis auf Null zu stellen, wie in Anspruch 20 angegeben.

Eine einfache Ausgestaltung der Sperrvorrichtung ist Gegenstand des Anspruchs 7, und die Ansprüche 8 und 9 betreffen eine einfache Anordnung des Führungsglieds.

Eine sehr einfache Ausgestaltung ist dadurch gekennzeichnet, daß das Führungsglied mit wenigstens einem Eingriffselement versehen ist, welches in der proximalen Endstellung des Stößels mit wenigstens einem hierzu im wesentlichen komplementären gehäusefesten Eingriffselement des Injektors in Eingriff steht. Durch das Eingriffselement wird das Führungsglied in der proximalen Endstellung des Stößels gegen Drehung arretiert und kann so die Gewindespindel in Längsrichtung im Gehäuse führen. Dabei geben die Ansprüche 11 bis 14 sehr vorteilhafte Ausgestaltungen für diese Arretierung an, die ja sehr sicher funktionieren und innerhalb kurzer Zeit wirksam werden muß.

Eine sehr vorteilhafte Weiterbildung der Erfindung ist dadurch gekennzeichnet, daß das Führungsglied dazu ausgebildet ist, beim Injektionsvorgang die Kartusche oder einen diese aufnehmenden Kartuschenträger durch direkte Anlage gegen diese bzw. diesen in proximaler Richtung zu verschieben. Hierdurch erreicht man eine sehr exakte Dosierung, besonders dann, wenn kleine Injektionsdosen injiziert werden.

Eine andere vorteilhafte Ausgestaltung zeichnet sich dadurch aus, daß die Sperrvorrichtung so ausgebildet ist, daß sie bei der Bewegung des Stößels von dessen distaler Endstellung zu dessen proximaler Endstellung mindestens so lange die Drehung des Stellglieds in der genannten umgekehrten Drehrichtung sperrt, wie das Führungsglied relativ zum Gehäuse frei verdrehbar ist. Dies ermöglicht eine sichere Einhaltung der eingestellten Injektionsdosis.

Eine andere vorteilhafte Ausgestaltung ist dadurch gekennzeichnet, daß das Verstellglied mit einem außerhalb des Gehäuses angeordneten Betätigungsorgan verbunden ist, welches die Verstellung des Stößels von dessen proximaler Endstellung in dessen distale Endstellung unter gleichzeitiger Spannung der dem Stößel zugeordneten Feder ermöglicht, und welches in der distalen Endstellung eine Verdrehung des Betätigungsorgans zur Vorwahl einer gewünschten Injektionsdosis ermöglicht. Dies ermöglicht eine einfache und sinnfällige Bedienung.

Eine weitere Lösung der gestellten Aufgabe ergibt sich erfindungsgemäß durch einen Injektor zur Aufnahme einer Kartusche mit einer gewöhnlich für eine Mehrzahl von Injektionen ausreichenden Menge an Injektionsflüssigkeit, welche Kartusche im Injektor gegen die Kraft einer Rückstellfeder in proximaler Richtung verschiebbar ist, mit einem durch eine Feder in proximaler Richtung beaufschlagten längenverstellbaren Stößel, welcher im Injektor zwischen einer proximalen Endstellung und einer distalen Endstellung verschiebbar ist und eine Gewindespindel aufweist, die im Gewinde eines Verstellglieds geführt ist, zur Beaufschlagung eines in der Kartusche vorgesehenen Kolbens dient, und der ein Führungsglied zugeordnet ist, das mit ihr drehfest, aber axial verschieblich, verbunden ist. Dieser Injektor ist dadurch gekennzeichnet, daß das Führungsglied dazu ausgebildet ist, bei der beim Injektionsvorgang erfolgenden proximalen Verschiebung der Kartusche diese, oder einen sie aufnehmenden Kartuschenträger, direkt zu beaufschlagen und zu verschieben, und daß das Führungsglied in der distalen Endstellung des Stößels relativ zum Gehäuse des Injektors verdrehbar ist, in der proximalen Endstellung aber nicht. Der formschlüssige Antrieb der Kartusche oder des Kartuschenträgers durch das Führungsglied bewirkt eine positive Verschiebung der Kartusche einerseits, gekoppelt mit einer Injektion erst dann, wenn die Injektionsnadel schon ins Gewebe des Patienten eingedrungen ist.

Eine bevorzugte Weiterbildung ergibt sich hierbei dadurch, daß zwischen dem Gehäuse und dem Verstellglied eine Feder vorgesehen ist, deren Vorspannung durch Verdrehen des Stellglieds veränderbar ist, und daß in der distalen Endstellung des Stößels eine Sperrvorrichtung vorgesehen ist, welche ein solches Verdrehen des Verstellglieds nur in einer bestimmten Richtung ermöglicht und eine Drehung in der umgekehrten Richtung sperrt, und/oder daß die Sperrvorrichtung in der proximalen Endstellung des Stößels nicht wirksam ist. Man kann so auch den Vorgang der Injektion in einfacher Weise automatisieren.

Eine sehr vorteilhafte Ausgestaltung ergibt sich hierbei dadurch, daß die Feder, deren Vorspannung durch Verdrehen des Verstellgliedes veränderbar ist, auch zum Beaufschlagen des längenverstellbaren Stößels in proximaler Richtung dient. Dadurch, daß eine Feder für zwei Funktionen verwendet wird, ergibt sich ein sehr einfacher Aufbau des Injektors.

In besonders bevorzugter Weise geht man dabei so vor, daß die zum Beaufschlagen des längenverstellbaren Stößels dienende Feder beim Injektionsvorgang zuerst Energie freigibt, die in ihr durch axiale Kompression gespeichert wurde, um eine axiale Kraft für das Einstechen der Nadel des Injektors zu bewirken, und anschließend Energie freigibt, welche in dieser Feder durch Verdrehung gespeichert wurde, um ein Drehmoment für die Injektion der zu injizierenden Flüssigkeitsmenge zu erzeugen. Auf diese Weise kann die Energie für den sequentiellen Ablauf - Einstechen der Nadel - Injizieren der eingestellten Dosis - einer einzigen Feder entnommen werden.

Weitere Einzelheiten und vorteilhafte Weiterbildungen der Erfindung ergeben sich aus dem im folgenden beschriebenen und in der Zeichnung dargestellten, in keiner Weise als Einschränkung der Erfindung zu verstehenden Ausführungsbeispiel, sowie aus den Unteransprüchen. Es zeigt:
- Fig. 1: eine raumbildliche Darstellung von inneren Elementen des Injektors vor deren Zusammenbau,
- Fig. 2: die Elemente der Fig. 1 nach deren Zusammenbau, in raumbildlicher Darstellung,
- Fig. 3: das Element der Fig. 2 zusammen mit weiteren Elementen des Injektors, vor deren Zusammenbau und in raumbildlicher Darstellung,
- Fig. 4: die Elemente der Fig. 3 nach deren Zusammenbau, in raumbildlicher Darstellung,
- Fig. 5: einen Längsschnitt durch das zusammengebaute Element der Fig. 4,
- Fig. 6: das Element der Figuren 4 und 5 zusammen mit weiteren Elementen des Injektors, vor deren Zusammenbau und in raumbildlicher Darstellung,
- Fig. 7: einen Längsschnitt durch das zusammengebaute Element der Fig. 6, welches dazu dient, bei einer Injektion zunächst die Nadel in den Körper des Patienten einzustechen und dann die zuvor eingestellte Dosis zu injizieren; das Element befindet sich in seiner nicht gespannten Grundstellung, wie sie auch in Fig. 17 dargestellt ist
- Fig. 8: einen Schnitt, gesehen längs der Linie VIII-VIII der Fig. 7, aber in einem gegenüber Fig. 7 vergrößerten Maßstab, um Einzelheiten besser erkennbar zu machen,
- Fig. 9: einen Schnitt, gesehen längs der Linie IX-IX der Fig. 7, aber in gegenüber Fig. 7 vergrößertem Maßstab, um Einzelheiten besser erkennbar zu machen,
- Fig. 10: einen Längsschnitt durch das Element der Fig. 7 im gespannten Zustand und nach Einstellen einer Injektionsdosis; dies ist dieselbe Stellung, wie sie auch in Fig. 18 dargestellt ist,
- Fig. 11 - 14: Schnitte, gesehen längs der Linien XI-XI bis XIV-XIV der Fig. 10,
- Fig. 15: eine raumbildliche Darstellung desjenigen Teils des Injektors, das zur Aufnahme einer (in Fig. 15 nicht dargestellten) Kartusche mit der zu injizierenden Flüssigkeit dient,
- Fig. 16: einen Längsschnitt durch die Teile der Fig. 15, in deren zusammengebautem Zustand,
- Fig. 17: eine Gesamtdarstellung eines erfindungsgemäßen Injektors in seiner Grundstellung, im Längsschnitt,
- Fig. 18: eine Gesamtdarstellung des Injektors der Fig. 17 in seiner gespannten Stellung und nach Einstellung einer Injektionsdosis; zur Verdeutlichung der Maßverhältnisse ist in Fig. 18 die Länge eines Zentimeters beispielhaft dargestellt,
- Fig. 19: eine Gesamtdarstellung des Injektors der Fig. 17 und 18 nach dem Einstich, aber vor dem Injizieren der Flüssigkeit, und
- Fig. 20: den Vorgang der Injektion der Injektionsflüssigkeit bei dem Injektor der Fig. 17 bis 19.

In der nachfolgenden Beschreibung werden die Begriffe proximal und distal in der in der Medizin üblichen Weise gebraucht, also proximal = dem Patienten zugewandt und distal = vom Patienten abgewandt. Die Begriffe links, rechts, oben, unten beziehen sich jeweils auf die betreffende Figur.

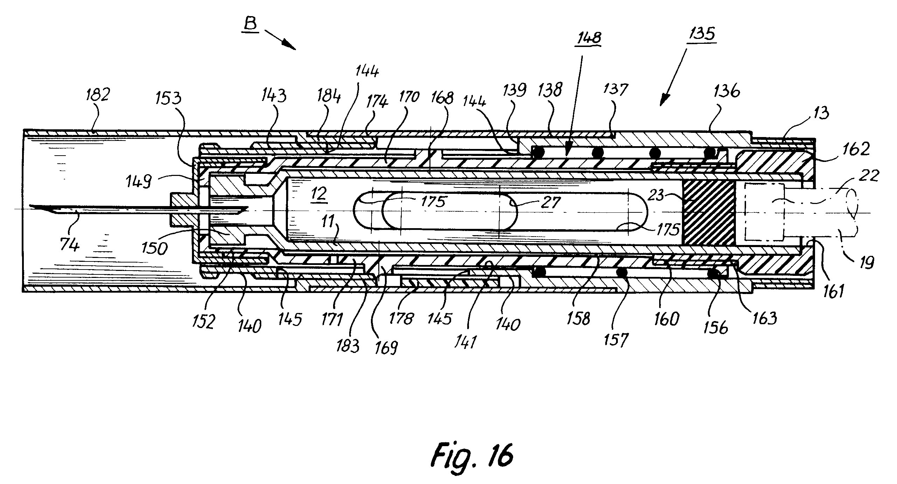

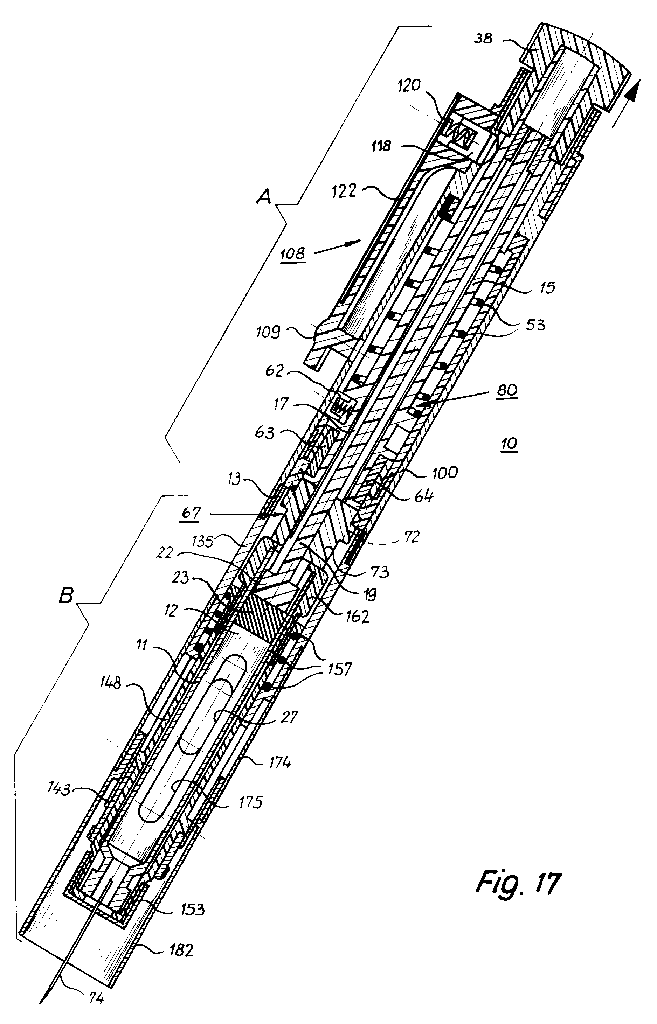

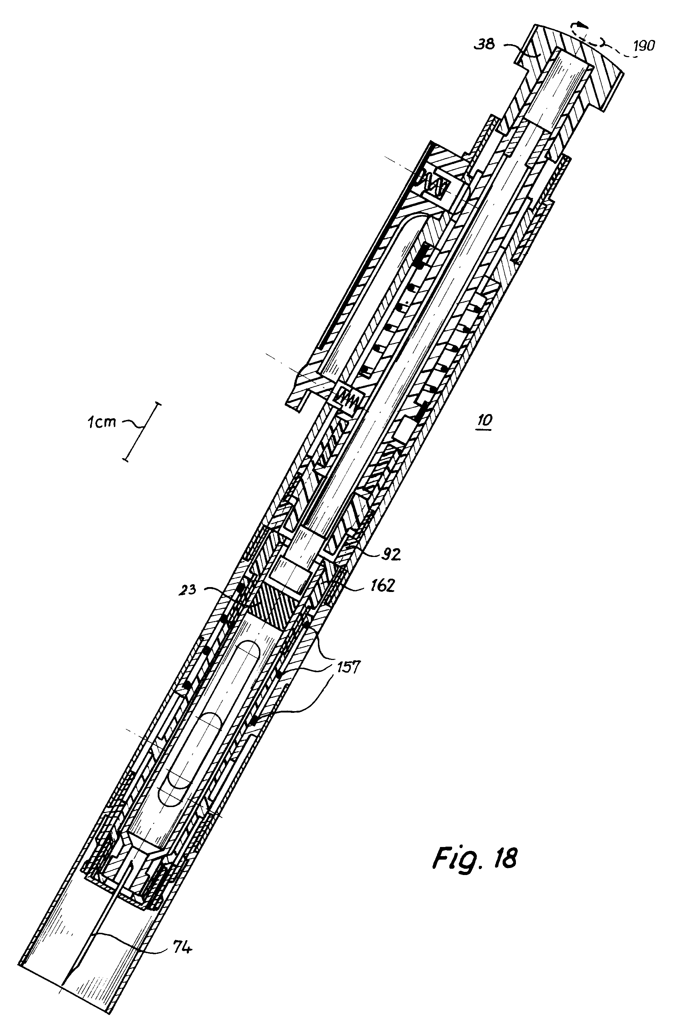

Die Fig. 17 bis 20 zeigen eine bevorzugte Ausführungsform eines fertig zusammengebauten, erfindungsgemäßen Injektors 10 in verschiedenen Betriebsstellungen. Fig. 18 gibt als Maßstab einen Zentimeter an. Dieser Maßstab gilt in gleicher Weise für die Fig. 16 und die Fig. 19 und 20. Das fertige Gerät hat etwa die Form eines überdimensionierten Füllfederhalters und in seiner Grundstellung gemäß Fig. 17 eine Gesamtlänge, gemessen ab der Nadelspitze von ca. 16,1 cm. Hinzu kommt eine (nicht dargestellte) Verschlußkappe für das proximale Injektorende mit der Injektionsnadel, so daß die Gesamtlänge bei ca. 17 cm liegen kann. Der Durchmesser des zylindrischen Teils kann z.B. 1,6 cm betragen. Es handelt sich also um ein sehr handliches Gerät; wie die folgende Beschreibung zeigen wird, ist auch seine Bedienung sehr einfach und weitgehend narrensicher.

Da die Zeichnungen nach den Fig. 17 bis 20 schwierig zu verstehen sind, wird im folgenden das Gerät etwa so beschrieben, wie es in der Fabrik aus seinen Einzelteilen montiert wird, wobei dann Aufbau und Funktion wesentlich anschaulicher werden.

Der dargestellte Injektor 10 hat einen distalen Teil A (Fig. 17), der zur Einstellung der zu injizierenden Dosis und zur Durchführung des Injektionsvorgangs dient, und er hat einen proximalen Teil B, der eine Kartusche 11 mit der zu injizierenden Flüssigkeit 12 aufnimmt. Die Teile A und B sind mittels eines Gewindes 13 zusammengeschraubt und können folglich auseinandergeschraubt werden, z.B., um eine leere Kartusche 11, deren Inhalt 12 verbraucht ist, durch eine volle Kartusche zu ersetzen, und um das Gerät für eine neue Kartusche entsprechend einzustellen.

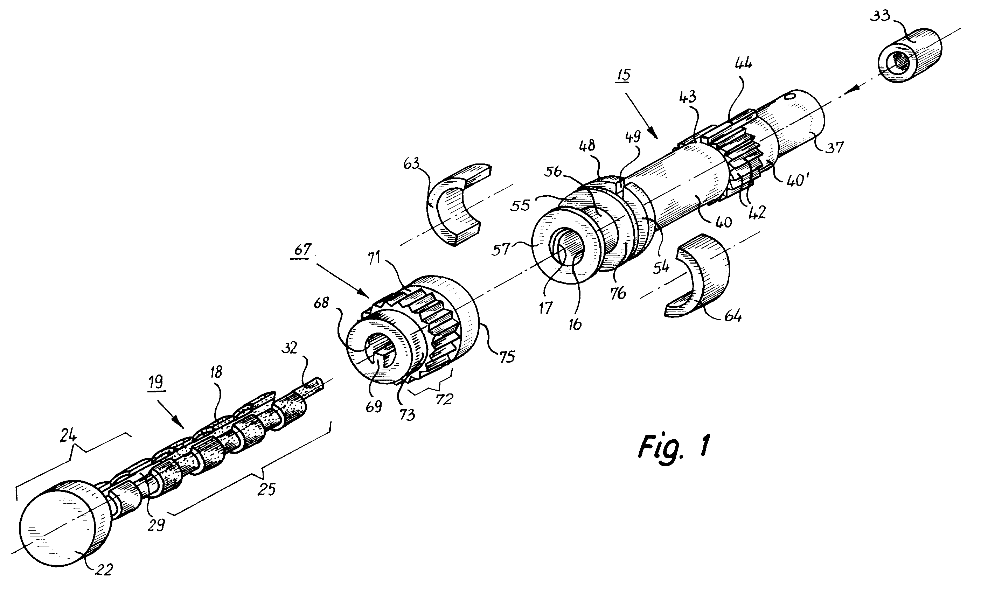

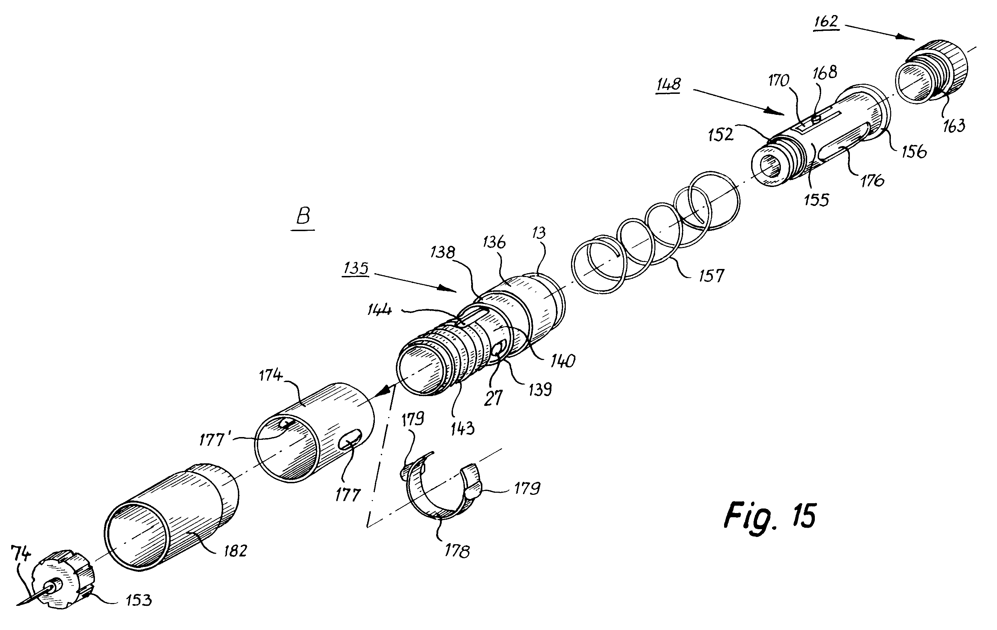

Im folgenden wird zunächst anhand der Fig. 1 bis 14 der Aufbau des Teils A beschrieben, und dann anhand der Fig. 15 und 16 der Aufbau des Teils B.

### Der A-Teil (Fig. 1 bis 14)

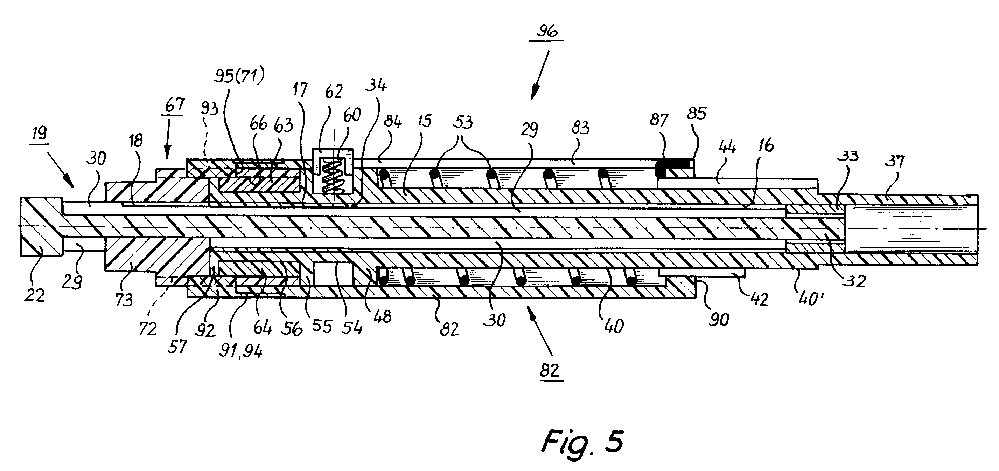

Fig. 1 zeigt ein Teil 15, das bei der Einstellung der Injektionsdosis verdreht wird und das deshalb als Verstellhülse bezeichnet wird. Wie man Fig. 5 entnimmt, hat es eine durchgehende zylindrische Ausnehmung 16, an deren linkem Endabschnitt ein mit 17 bezeichnetes Innengewinde vorgesehen ist, das dazu dient, ein Außengewinde 18 einer Gewindespindel 19 aufzunehmen, deren freies, proximales Ende 22 etwas verbreitert sein kann und zur Anlage gegen einen Kolben 23 (Fig. 17) der Kartusche 11 dient, welcher Kolben gewöhnlich aus einem geeigneten Gummi besteht und hier nur schematisch dargestellt ist.

Das Innengewinde 17 bzw. das zu ihm komplementäre Außengewinde 18 ist bevorzugt ein Rechteck-Steilgewinde mit etwa zwei Gewindegängen pro Zentimeter bei einem Gewinde-Außendurchmesser von 0,5 cm. Wie in Fig. 1 dargestellt, ist die Gewindespindel zweifarbig ausgeführt. Ihr vorderer, proximaler Teil 24 kann z.B. auf eine Länge von 1,5 cm weiß sein, also gemessen ab dem proximalen Ende der Gewindespindel 19. Ihr übriger Teil 25 hat eine andere Farbe, z.B. rot, was in Fig. 1 durch Punkte angedeutet ist.

Wie Fig. 17 zeigt, hat das Teil B ein Fenster 27, durch das der Benutzer den Inhalt (Füllzustand) der Kartusche 11 überwachen kann. Wenn in diesem Fenster 27 der rote Teil der Gewindespindel 19 voll sichtbar wird, weiß der Benutzer, daß es nun Zeit ist, die Kartusche zu wechseln. In Fig. 17 ist z.B. die Gewindespindel 19 noch gar nicht im Fenster 27 sichtbar, d.h. die Kartusche 11 ist voll, aber nach mehreren Injektionen nähert sich der Kolben 23 immer mehr dem proximalen Ende der Kartusche 11, und entsprechend wird die Gewindespindel 19 immer mehr aus der Verstellhülse 15 herausgeschraubt und wird dann im Fenster 27 sichtbar. (In Fig. 1 ist die Gewindespindel 19 nur verkürzt dargestellt; Fig. 5 zeigt ihre tatsächliche Länge. Aus Fig. 5 erkennt man auch, daß das Außengewinde 18 der Gewindespindel 19 erst in einem bestimmten Abstand von deren proximalem Ende zu beginnen braucht.)

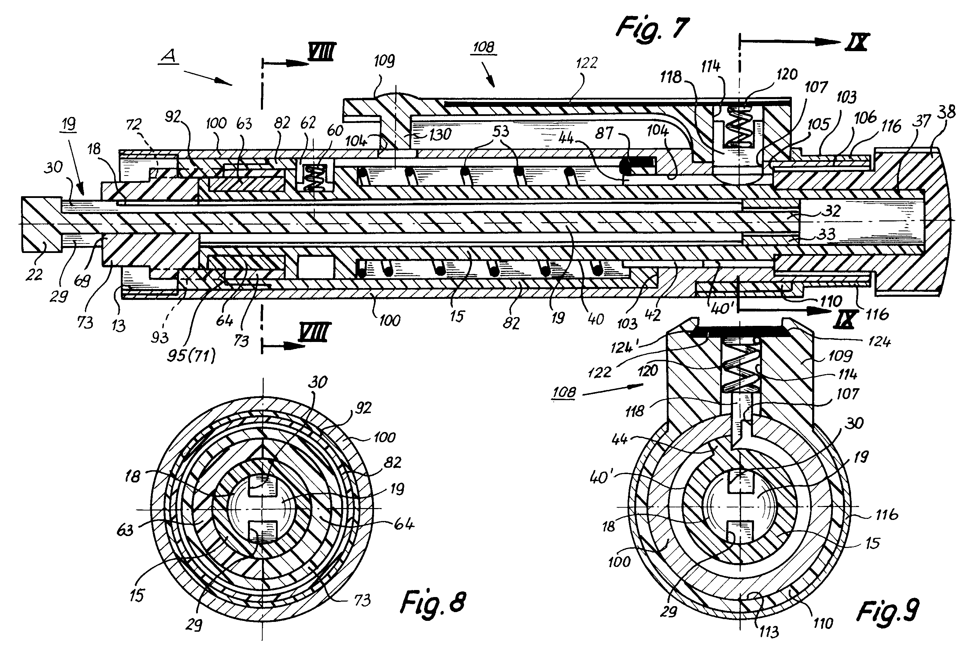

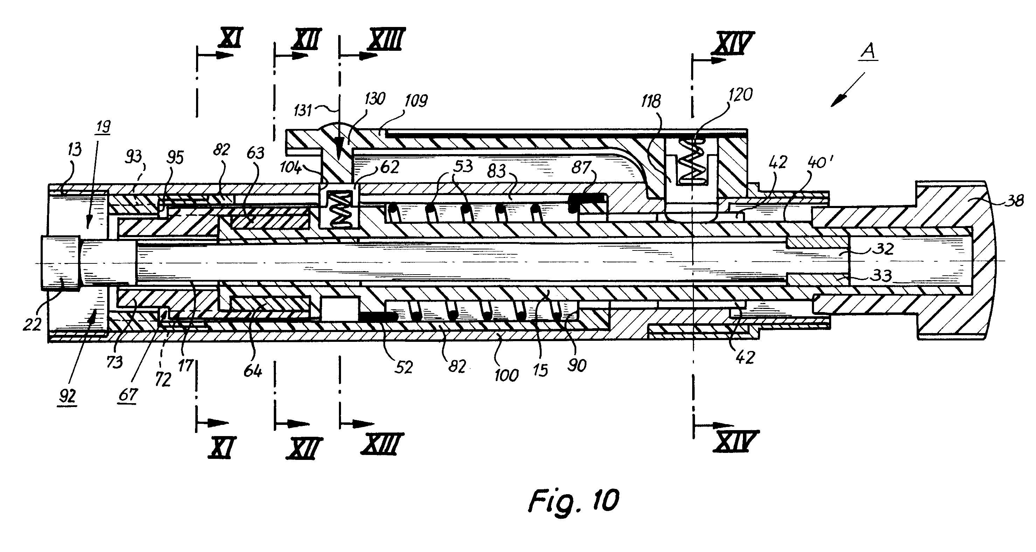

Zusätzlich zum Außengewinde 18 hat die Gewindespindel 19 zwei diametral gegenüberliegende Längsnuten 29, 30, vgl. z.B. die Fig. 8 und 9. An sich würde eine einzige Längsnut, z.B. die Längsnut 29, genügen, aber da die Gewindespindel 19 bevorzugt aus Kunststoff hergestellt wird, ist es günstiger, zwei symmetrische Längsnuten 29, 30 vorzusehen, da sich dann die Gewindespindel 19 nicht "verziehen" kann, also nicht krumm wird, wenn sie nach dem Kunststoffspritzen (moulding) aus ihrer Form herausgenommen wird.

Die Gewindespindel 19 hat an ihrem distalen Ende einen Zapfen 32 reduzierten Durchmessers, auf dem nach der Montage eine zylindrische Hülse 33 befestigt wird, z.B. durch Kleben oder durch Warmverformung. Diese Hülse 33 verhindert, wenn sie an das distale Ende 34 (Fig. 5) des Innengewindes 17 stößt, daß die Gewindespindel 19 noch weiter aus der Verstellhülse 15 herausgedreht wird, wirkt also als Anschlag.

Verdreht man die Verstellhülse 15 und die Gewindespindel 19 relativ zueinander, so wird das so gebildete Gesamtteil, das man auch als Stößel bezeichnen kann, je nach relativer Drehrichtung kürzer oder länger. Vor jeder Injektion wird eingestellt, um wieviel dieses Gesamtteil bei einer Injektion länger werden soll, und dadurch wird vor der Injektion die Injektionsdosis festgelegt.

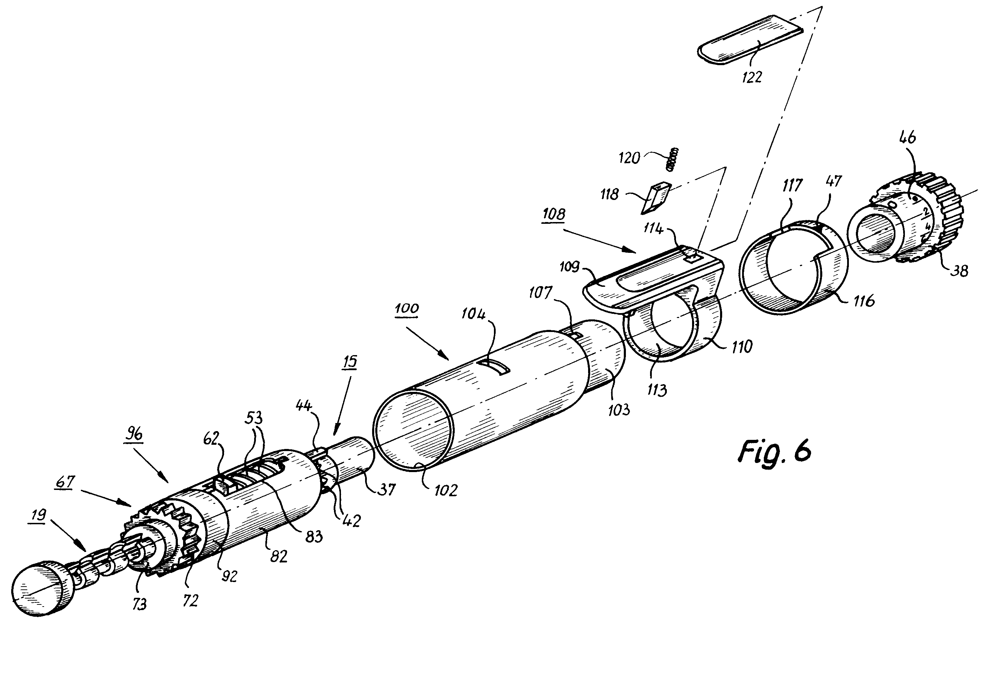

Die Verstellhülse 15 hat an ihrem distalen Ende einen zylindrischen Abschnitt 37 reduzierten Durchmessers, auf dem nach der Montage ein Betätigungsknopf 38 (vgl. z.B. die Fig. 6 und 7) montiert wird, z.B. mittels einer Schraube, durch eine Schnappverbindung, oder durch Festkleben. An den zylindrischen Abschnitt 37 schließt sich in proximaler Richtung ein zylindrischer Abschnitt 40 etwas größeren Durchmessers an, in dessen distalem Bereich Ratschenzähne 42 vorgesehen sind, deren Schnittdarstellung Fig. 14 zeigt. Beim Ausführungsbeispiel handelt es sich um 18 Zähne 42 gleichen Abstands, was also die Einstellung von 18 verschiedenen Injektionsdosen ermöglicht. Diese Zähne nehmen etwa 330° des Umfangs der Verstellhülse 15 ein. In der Lücke zwischen ihnen befinden sich eine Längsnut 43 und ein verbreiterter Anschlag 44 mit rechteckförmigem Querschnitt, der sich in distaler Richtung über die Ratschenzähne 42 hinaus und bis zum distalen Ende des zylindrischen Abschnitts 40 erstreckt. Anders gesagt, befindet sich in Fig. 1 rechts von den Ratschenzähnen 42 ein zahnfreier Abschnitt 40' des Abschnitts 40, in den sich der verbreiterte Anschlag 44 erstreckt. Man erkennt das völlig klar in Fig. 1.

Die Zähne 42 selbst sind bevorzugt so ausgebildet, daß sie, wenn man auf das distale Ende des Betätigungsknopfes schaut, eine Drehung dieses Knopfes 38 im Uhrzeigersinn erlauben, nicht aber entgegen dem Uhrzeigersinn. Der Betätigungsknopf 38 kann mit einer Skala 46 zur Einstellung der Injektionsdosis versehen sein, vgl. Fig. 6, und dieser Skala entspricht am Gehäuse eine feststehende Skalenmarke 47, vgl. Fig. 6.

Alternativ kann naturgemäß die Skalenmarke am Betätigungsknopf 38, der als Stellglied dient, vorgesehen werden, während dann in diesem Fall die Skala 46 am feststehenden Gehäuseteil 116 angebracht wird, was den Vorteil hat, daß dort die Zahlen der Skala 46 größer und dadurch besser lesbar sein können. Diese Variante ist in der Zeichnung nicht dargestellt.

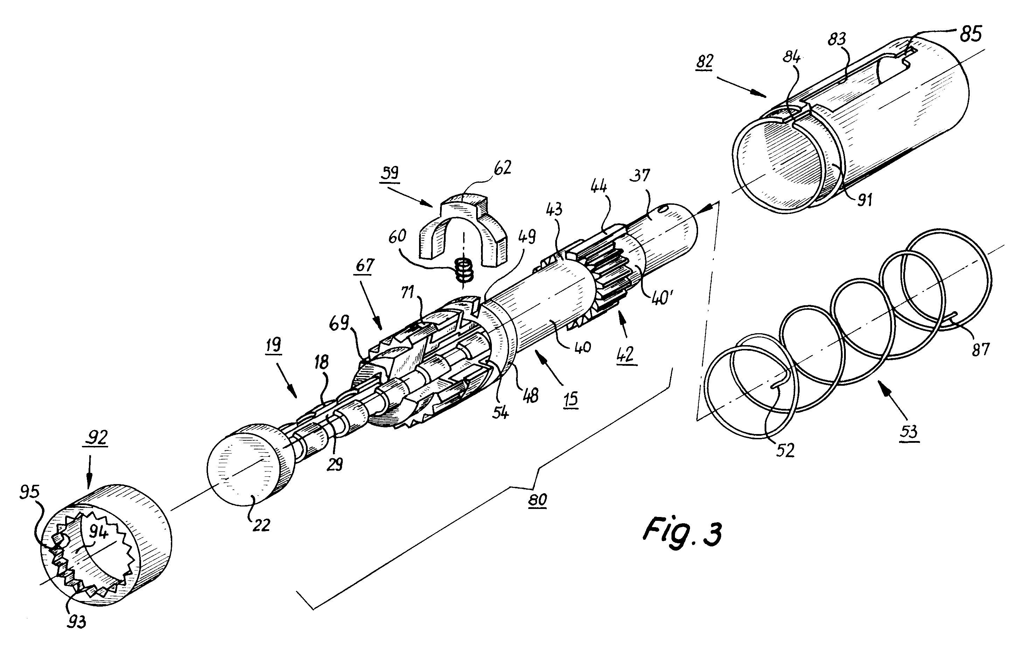

Am proximalen Ende des zylindrischen Abschnitts 40 geht dieser über in einen radial abstehenden Flanschabschnitt 48, der mit einer Unterbrechung zur Aufnahme des proximalen Endes 52 einer Schraubenfeder 53 (Fig. 3) versehen ist. Vom Flanschabschnitt 48 durch eine Ringnut 54 getrennt ist ein Flanschabschnitt 55, und von diesem durch eine Ringnut 56 (Fig. 1) getrennt ist ein Flanschabschnitt 57 am proximalen Ende der Verstellhülse 15, welch letztere bevorzugt als Spritzgußteil aus einem geeigneten Kunststoff ausgebildet ist, vgl. die Fig. 1 und 5.

Wie Fig. 3 zeigt, dient die Ringnut 54 zur Aufnahme eines Rastglieds 59, das etwa wie ein auf dem Kopf stehendes U ausgebildet ist und das durch eine kleine Spiralfeder 60 aus dieser Ringnut 54 radial nach außen gedrückt wird. Sein eigentlicher Rastzapfen ist mit 62 bezeichnet.

Die Ringnut 56 (Fig. 1) dient zur Aufnahme zweier federnder Halbschalen 63, 64 aus Kunststoff, über die, wenn sie sich in der Ringnut 56 befinden, eine zylindrische Innenausnehmung 66 (Fig. 5) eines Führungsgliedes 67 geschoben und dabei bevorzugt mit den Halbschalen 63, 64 verklebt wird, so daß die Halbschalen 63, 64 das Führungsglied 67 am proximalen Ende der Verstellhülse 15 festhalten.

Die federnden Halbschalen 63, 64 haben eine Mehrfachfunktion:
a) Sie halten das Führungsglied 67 am proximalen Ende der Verstellhülse 15 sicher fest, d.h. für das Abziehen des Führungsglieds 67 ist z.B. eine Kraft von einem Dutzend N oder mehr erforderlich.
b) Sie ermöglichen andererseits eine sehr einfache Montage des Führungsglieds 67.
c) Sie ermöglichen eine Verdrehung des Führungsglieds 67 relativ zur Verstellhülse 15, wobei diese Verdrehung, durch die Reibungswirkung der federnden Halbschalen 63, 64, im Rahmen der Erfindung nicht allzuleicht möglich sein soll, und diese Halbschalen 63, 64 können deshalb so ausgebildet werden, daß sie diese Verdrehung im gewünschten Maße bremsen. Sie können z.B. auf ihrer Innenseite mit kleinen Vorsprüngen versehen sein, die in entsprechende Rastzähne (nicht dargestellt) am Boden der Ringnut 56 eingreifen, oder ähnlich.

Naturgemäß könnte das Führungsglied 67 auch in anderer Weise drehbar am proximalen Ende der Verstellhülse 15 befestigt werden, wozu dem Fachmann zweifellos sehr viele Möglichkeiten zu Gebote stehen. Die dargestellte Lösung wird aber wegen ihrer großen Einfachheit bevorzugt.

Das Führungsglied 67 hat eine axiale Ausnehmung 68 (Fig. 1) für die Gewindespindel 19, und diese axiale Ausnehmung 68 ist mit einer radial nach innen ragenden Führungsnase 69 versehen, die nach der Montage in die Längsnut 29 der Gewindespindel 19 eingreift, wie das Fig. 3 zeigt. Wird also die Gewindespindel 19 z.B. von Hand verdreht, so wird hierdurch auch das Führungsglied 67 verdreht, und wenn dieses gegen Verdrehung festgehalten wird, ist auch die Gewindespindel 19 gegen Verdrehung gesperrt, wie man ohne weiteres einsieht.

Diese Möglichkeit der freien Verdrehung der Gewindespindel 19 wird benutzt, um diese - nach dem Laden einer neuen Kartusche 11 - von Hand vollständig in die Verstellhülse 15 zurückzudrehen, d.h. noch etwas weiter, als das in Fig. 5 dargestellt ist. Hierzu wird die Verstellhülse 15 festgehalten, während das Führungsglied 67 frei verdrehbar ist.

Zur Einstellung der Dosis wird, wie nachfolgend beschrieben werden wird, die Gesamtheit von Verstellhülse 15, Führungsglied 67 und Gewindespindel 19 gleichsinnig vorwärts verdreht, d.h. alle drei Teile drehen sich hierbei um denselben, vom Benutzer gewünschten Winkel, z.B. um 30°. Die beiden Enden 52, 87 der Feder 53 werden dabei relativ zueinander verdreht, und die Feder 53 wird dadurch auf Torsion gespannt, bzw. ihre Torsions-Vorspannung wird hierdurch erhöht.

Wenn hier gesagt wird, daß die Gesamtheit der angegebenen Teile "vorwärts" verdreht wird, bedeutet das beim Ausführungsbeispiel eine Verdrehung im Uhrzeigersinn, wenn man auf das distale Ende des Injektors 10 schaut. Ebenso bedeutet nachfolgend "rückwärts" eine entsprechende Verdrehung entgegen dem Uhrzeigersinn.

Anschließend wird zur Injektion der eingestellten Flüssigkeitsmenge das Führungsglied 67 in seiner proximalen Endstellung (Fig. 7) im Gehäuse des Injektors 10 gegen Verdrehung festgehalten, und die Verstellhülse 15 wird um den zuvor eingestellten Winkel, z.B. die angegebenen 30°, durch die Torsionskraft der Feder 53 rückwärts gedreht, wodurch die Gewindespindel 19 um einen entsprechenden Weg aus dem Innengewinde 17 herausgedreht wird und dabei den Kolben 23 der Kartusche 11 entsprechend weit verschiebt und die eingestellte Flüssigkeitsdosis aus der Kartusche auspreßt.

Aus der nachfolgenden Beschreibung werden diese Abläufe, die in der Praxis vollautomatisch und sehr rasch ablaufen, dem Leser noch klarer werden.

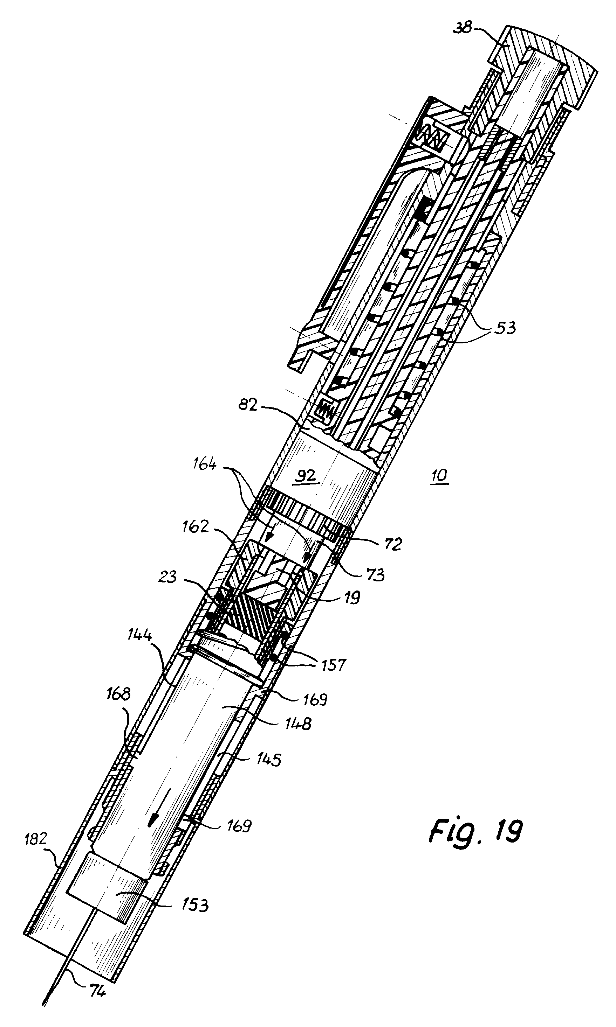

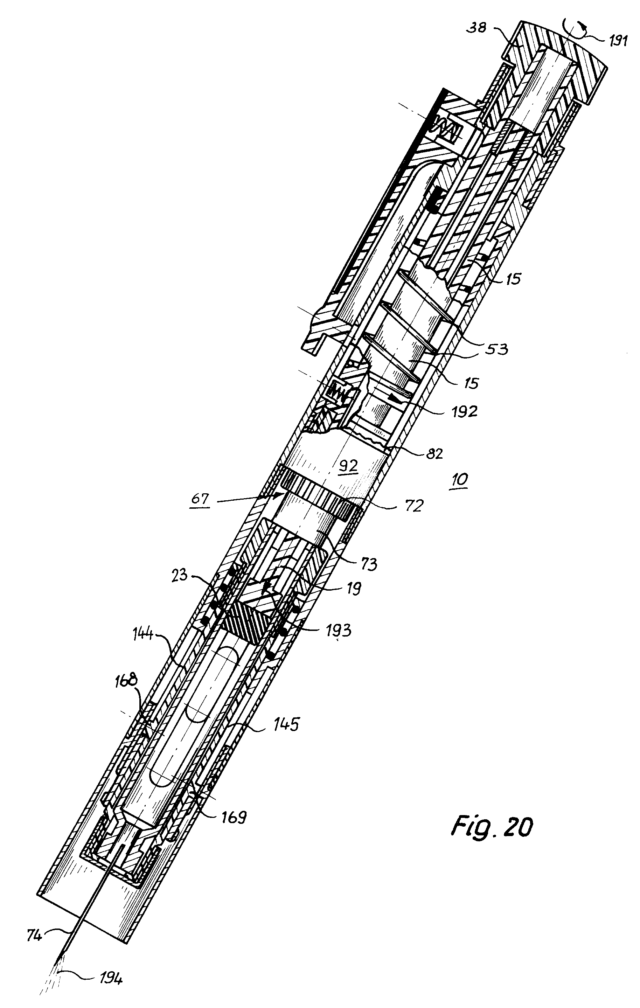

Das Führungsglied 67 ist auf seinem Außenumfang mit einer Axialverzahnung 72 versehen, die sich in distaler Richtung bis zu einer Schulter 71 erstreckt. In der Praxis kann der Durchmesser dieser Axialverzahnung in proximaler Richtung etwas abnehmen, d.h. die Höhe der Zähne kann z.B. in proximaler Richtung um etwa 0,5 bis 1 % abnehmen. Die Verzahnung sieht in diesem Fall ganz leicht kegelstumpfförmig (frustokonisch) aus. Dies erleichtert ihre Funktion. An den Abschnitt 72 mit der Axialverzahnung schließt sich ein zylindrischer Abschnitt 73 kleineren Durchmessers an, dessen Funktion es ist, beim Injektionsvorgang die Kartusche 11 und eine an ihr befestigte Injektionsnadel 74 in proximaler Richtung zu verschieben und dadurch die Nadel 74 in den Patienten einzustechen. Dieser Vorgang ist in Fig. 19 dargestellt. Er geht der Injektion der eingestellten Flüssigkeitsmenge voraus, wie sie in Fig. 20 dargestellt ist.

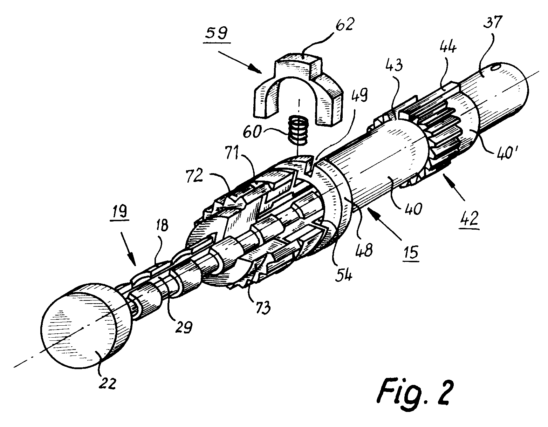

Bei der Montage der in Fig. 1 dargestellten Teile werden zunächst die Halbschalen 63, 64 in die Ringnut 56 eingesetzt, und anschließend wird das Führungsglied 67 mit seiner Innenausnehmung 66 über diese Halbschalen 63, 64 gepreßt, so daß es die Lage gemäß Fig. 2 einnimmt und auf der Verstellhülse 15 verdreht werden kann, jedoch relativ zu dieser nicht verschiebbar ist, da es mit seinem distalen Rand 75 gegen die proximale Seite 76 des Flansches 55 anliegt und zusammen mit diesem ein Axial-Gleitlager bildet (vgl. Fig. 1).

Wenn das Führungsglied 67 montiert ist, wird das distale Ende der Gewindespindel 19 in die Öffnung 68 geschoben, bis dieses Ende zum Eingriff mit dem Innengewinde 17 der Verstellhülse 15 kommt, und wird dann in dieses Innengewinde 17 eingeschraubt. Anschließend wird die zylindrische Hülse 33 (Fig. 5) auf dem Zapfen 32 der Gewindespindel 19 befestigt, so daß es dann nicht mehr möglich ist, die Gewindespindel 19 vollständig aus dem innengewinde 17 herauszuschrauben und die Hülse 33 bei einem derartigen Versuch als Anschlag wirkt.

Das montierte Teil, das man wie gesagt auch als Stößel 80 bezeichnen kann, hat dann die in Fig. 3 dargestellte Form.

Gemäß Fig. 3 wird anschließend das bereits beschriebene Rastglied 59 samt Feder 60 in die Ringnut 54 eingesetzt, und es wird dann die Feder 53 über das distale Ende dieses Stößels 80 geschoben, wobei das proximale Ende 52 der Feder 53 in die Aussparung 49 eingreift und folglich ein Drehmoment von der Feder 53 auf die Verstellhülse 15 übertragen kann, ebenso eine axiale Kraft, wenn die Feder 53 entsprechend gespannt ist.

Es ist hier der Ort, darauf hinzuweisen, daß die Feder 53 beim Ausführungsbeispiel nicht nur zur Erzeugung einer axialen Kraft auf die Verstellhülse 15 dient, sondern auch zur Erzeugung eines Drehmoments, mit dem Ziel der Verdrehung dieser Verstellhülse.

Nach der Montage der Feder 53 wird eine Hülse 82 aus Kunststoff vom distalen Ende her über die Verstellhülse 15 geschoben. Diese Hülse 82 hat in ihrem Mittelbereich eine relativ breite axiale Längsnut 83, die sich am proximalen Ende in einer schmalen Längsnut 84 und am distalen Ende in einer schmalen Längsnut 85 fortsetzt.

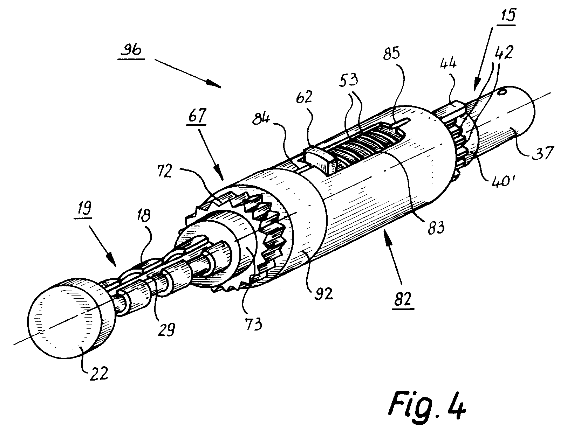

Die breite Längsnut 83 dient zur Aufnahme des Rastzapfens 62 des Rastglieds 59, d.h. dieser Rastzapfen 62 ragt, wie in Fig. 4 dargestellt, aus der breiten Längsnut 83 heraus und kann entgegen der Wirkung seiner Feder 60 radial nach innen gedrückt werden.

Das distale Ende 87 der Feder 53 ragt in die schmale Längsnut 85 der Hülse 82 und ist dort gegen Verdrehung und axial-distale Verschiebung gesichert. Dies ist auch in Fig. 5 sehr anschaulich dargestellt.

An ihrem distalen Ende hat die Hülse 82 einen radial nach innen ragenden Flansch 90, mit dem sie auf den Ratschenzähnen 44 gleiten kann, vgl. Fig. 5. Dieser Flansch 90 dient als Anlage für den distalen Teil der Feder 53. An ihrem proximalen Ende hat die Hülse 82 einen Abschnitt 91 reduzierten Durchmessers.

Auf diesem Abschnitt 91 wird vom proximalen Ende her ein hülsenartiges Teil 92 aufgeschoben, das in seinem proximalen Bereich mit einer zur Außenverzahnung 72 komplementären Innenverzahnung 93 versehen ist, während es in seinem distalen Bereich eine zylindrische Innenausnehmung 94 aufweist, die etwa dem Außendurchmesser des Abschnitts 91 entspricht und auf diesen - vorzugsweise nur in einer bestimmten Drehstellung, welche durch den Eingriff einer (nicht sichtbaren) Nase des Teil 92 in die schmale Längsnut 84 definiert ist - aufgeschoben werden kann, wobei zwischen die Abschnitte 91 und 94 etwas Klebstoff gebracht wird und diese Abschnitte dadurch miteinander verklebt werden. Durch die axiale Kraft der gespannten Feder 53 wird in diesem Fall das Führungsglied 67 mit seiner Außenverzahnung 72 in die Innenverzahnung 93 des hülsenartigen Teils 92 gepreßt und ragt, wie in Fig. 4 und 5 dargestellt, mit seiner Außenverzahnung 72 etwas aus dieser Innenverzahnung 93 heraus.

Eine Schulter 95 (Fig. 5) am distalen Ende der Innenverzahnung 93 wirkt bei der Injektion als axialer Anschlag für die Schulter 71 (Fig. 1) am distalen Ende der Außenverzahnung 72 und definiert so präzise die proximale Endstellung des Führungsglieds 67 relativ zum Teil 92.

Durch diese Montagevorgänge hat man also das Teil 96 gemäß Fig. 4 bzw. 5 erhalten. Hält man in Fig. 4 die Hülse 82 des Teiles 96 fest und dreht am Abschnitt 37 der Verstellhülse 15, so wird die Gewindespindel 19 entweder aus der Verstellhülse 15 heraus- oder in diese hineingedreht, je nach Drehrichtung. Dabei dreht sich das Rastglied 59 in der Ringnut 54, während das Führungsglied 67 durch die Innenverzahnung 93 gegen Verdrehung relativ zur Hülse 92 gesichert ist.

Es soll hier darauf hingewiesen werden, daß das Führungsglied 67 in seiner proximalen Endstellung a) eine vorgegebenen axiale Lage relativ zum Gehäuse einnehmen
und
b) gegen Drehung blockiert sein muß.
Zur Erreichung dieser Ziele gibt es naturgemäß vielerlei Möglichkeiten. So wäre es z.B. auch möglich, daß das Führungsglied 67 in seiner proximalen Endstellung einfach mit einer proximalen Schulter (analog der Schulter 71) gegen eine entsprechende distale Schulter des Teils 92 (analog der Schulter 95) mit genügend Reibung anliegt, oder daß an diesen Schultern entsprechende Vorsprünge bzw. dazu komplementäre Vertiefungen vorgesehen werden, die in dieser Lage eine Verdrehung des Führungsglieds 67 relativ zum Teil 92 sicher verhindern. Diese Möglichkeit ist in der Zeichnung nicht separat dargestellt.

Das Teil 96 in seiner Form gemäß Fig. 4 und 5 ist nun für die Montage im Gehäuse des Injektors 10 bereit.

Wie Fig. 6 zeigt, hat dieses Gehäuse ein Teil in Form eines Rohres 100, z.B. aus Aluminium, mit einer zylindrischen Ausnehmung 102, die an den Außendurchmesser des Teils 96 angepaßt ist und dieses ohne Spiel aufnehmen kann. Eine Ringschulter 103 im distalen Teil des Rohres 100 wirkt bei der Montage als Anschlag für das distale Ende der Hülse 82, vgl. Fig. 7.

Das Teil 96 wird so in die zylindrische Ausnehmung 102 des Rohres 100 eingesetzt, daß eine rechteckförmige seitliche Öffnung 104 des Rohres 100 mit der verbreiterten Längsnut 83 fluchtet. Wird also die Verstellhülse 15 durch Ziehen in distaler Richtung verschoben, wobei die Feder 53 gespannt wird, so kann der Rastzapfen 62 in diese Öffnung 104 einrasten, wie das in Fig. 10, 13 und 18 dargestellt ist. Dies ist die Spannstellung des Injektors 10, in die er vor einer Injektion gebracht werden muß, damit überhaupt eine Dosiseinstellung möglich ist. In dieser Stellung ist die Feder 53 gespannt und speichert dadurch die Energie, die zum Einstechen der Nadel 74 in den Patienten notwendig ist.

Das Teil 96 wird also in dieser Stellung in das Rohr 100 eingesetzt, und in dieser Stellung wird die Hülse 82 im Rohr 100 dauerhaft befestigt, z.B. durch Kleben, mittels einer Schraube, oder in sonstiger geeigneter Weise.

Das Rohr 100 hat an seinem distalen Ende einen äußeren, zylindrischen Abschnitt 103 reduzierten Durchmessers, und es hat in seinem Inneren einen zylindrischen Abschnitt 104 reduzierten Durchmessers, der sich von der Schulter 103 weg in distaler Richtung erstreckt bis zu einer Schulter 105, wo dieser Abschnitt 104 in einen zylindrischen Abschnitt 106 etwas größeren Innendurchmessers übergeht, der sich bis zum distalen Ende des Rohres 100 erstreckt (Fig. 7).

Nach der Montage des Teils 96 wird auf den zylindrischen Abschnitt 103, der mit einer schmalen radialen Ausnehmung 107 versehen ist, ein Clip 108 aufgeschoben, der einen federnden Clipabschnitt 109 und einen ringförmigen Abschnitt 110 aufweist. Eine zylindrische Ausnehmung 113 des Abschnitts 110 paßt ohne Spiel auf den zylindrischen Abschnitt 103. Der Clip 108 hat eine radial verlaufende Ausnehmung 114, die im montierten Zustand mit der radialen Ausnehmung 107 des Rohres 100 fluchtet. In dieser fluchtenden Stellung wird der ringförmige Abschnitt 110 auf dem zylindrischen Abschnitt 103 befestigt, z.B. durch Kleben. Alternativ kann er auf diesen Abschnitt aufgepreßt werden.

Anschließend wird eine rohrförmige Blende 116, welche eine rechteckförmige Aussparung 117 für den Clip 109 hat, über den zylindrischen Abschnitt 103 des Rohre 100 und die Außenseite des ringförmigen Abschnitts 110 geschoben und in dieser Stellung befestigt, z.B. durch Verkleben. Die genaue Form dieser Blende 116 ergibt sich z.B. aus Fig. 7.

Nach Befestigen der Blende 116 kann der Drehknopf 38 auf dem Abschnitt 37 der Verstellhülse 15 befestigt werden, wie das bereits beschrieben wurde.

Zum Eingriff in die Ratschenzähne 42 bzw. mit dem Anschlag 44 ist eine Klinke 118 vorgesehen, deren Querschnittsform aus den Fig. 7 und 9 klar hervorgeht. Dabei sei nochmals darauf hingewiesen, daß - zum besseren Verständnis der Erfindung - die Darstellungen der Fig. 8 und 9 gegenüber Fig. 7 vergrößert sind.

Die Klinke 118 wird einfach von oben in die Ausnehmungen 114, 107 eingesetzt, aber erst, nachdem der Drehknopf 38 um einen bestimmten Winkel so weit im Uhrzeigersinn (von der distalen Seite des Injektors 10 her gesehen) verdreht worden ist, bis die Feder 53 auf ein vorgegebenes Drehmoment vorgespannt worden ist. Gewöhnlich genügt hierfür eine halbe Umdrehung des Drehknopfs 38. In dieser gespannten Stellung läßt man die Klinke 118 - wie ein Fallbeil (Guillotine) - in die Ausnehmungen 114, 107 fallen, so daß sie mit ihrem inneren Ende auf den Abschnitt 40' des zylindrischen Abschnitts 40 zu liegen kommt (Fig. 7 und 9).

Nun kann man den Drehknopf 38 loslassen, und durch die Vorspannung der Feder 53 legt sich der Vorsprung bzw. Anschlag 44 so gegen diese Klinke 118, wie das Fig. 9 zeigt.

Oberhalb der Klinke 118 wird eine kleine Schraubenfeder 120 montiert, und über dieser wird eine Abdeckplatte 122 in zwei zueinander parallele Nuten 124, 124' des Clipabschnitts 109 eingeschoben und bewirkt so eine entsprechende Vorspannung der Feder 120 (Fig. 9).

Der A-Teil des Injektors 10 ist nun fertig montiert und benutzungsfertig. Es ist ein Wunder der Feinmechanik, weil er eine außerordentlich einfache Benutzung ermöglicht.

### Arbeitsweise des A-Teils

Fig. 7 zeigt den A-Teil in der Grundstellung, und Fig. 10 in seiner gespannten Stellung nach Einstellung einer Injektionsdosis.

Was geschieht, wenn man versucht, in der Grundstellung gemäß Fig. 7 eine Injektionsdosis einzustellen?

In diesem Fall befindet sich die Außenverzahnung 72 des Führungsglied 67 in Eingriff mit der Innenverzahnung 93, also mit dem Gehäuse des Injektors. Das Führungsglied 67 kann sich folglich in dieser Stellung nicht drehen.

Der Anschlag 44 (vgl. z.B. Fig. 9) ermöglicht eine Drehung des Betätigungsknopfes 38 nur im Uhrzeigersinn, gesehen vom distalen Ende des Injektors 10. Eine solche Drehung hat zur Folge, daß die Gewindespindel 19 in die Verstellhülse 15 hineingeschraubt wird, d.h. der in Fig. 3 dargestellte Stößel 80 wird hierbei kürzer und nicht länger. - Bei einer solchen Drehung des Betätigungsknopfes 38 wird die Feder 53 zusätzlich auf Torsion gespannt. Läßt man den Betätigungsknopf 38 los, so dreht die Feder 53 den Betätigungsknopf 38 wieder in seine Ausgangsstellung zurück, in welcher der Anschlag 44 gegen die Klinke 118 anliegt, wie das Fig. 9 zeigt, und die Gewindespindel 19 nimmt ebenfalls wieder ihre vorherige Lage ein.

Es ist also festzuhalten, daß ein Versuch einer Dosiseinstellung in der Grundstellung (Fig. 7) ohne Resultat bleibt, und daß hierbei der Stößel 80 (Fig. 3) nicht länger, sondern kürzer wird.

Dieser Umstand ermöglicht es, einen sehr kurzen Injektor zu bauen, da in der Grundstellung eine Vergrößerung der Länge des Stößels 80 (Fig. 3) unmöglich ist und man folglich hierfür keine Sicherheitszuschläge vorsehen muß.

Wird das A-Teil gespannt, wie das in Fig. 10 dargestellt ist, so rastet der Rastzapfen 62 in die Ausnehmung 104 des Rohres 100 ein, und die Feder 53 erhält durch diesen Spannvorgang eine erhöhte Vorspannung.

Wie Fig. 10 klar zeigt, kommt in dieser Stellung die Außenverzahnung 72 des Führungsglieds 67 außer Eingriff mit der Innenverzahnung 93 des Teils 92, so daß sich nun das Führungsglied 67 frei und unbehindert im Inneren der Hülse 82 drehen kann. Eine solche reibungsfreie und unbehinderte Drehung ist notwendig für eine richtige Einstellung der Injektionsdosis, damit beim Einstellvorgang eine relative Drehung zwischen der Verstellhülse 15 und dem Führungsglied 67 sicher vermieden wird. Aus diesem Grunde ist es auch vorteilhaft, wenn, wie eingangs beschrieben, für eine solche relative Drehung ein bestimmtes Mindestdrehmoment erforderlich ist.

In der Stellung gemäß Fig. 10 greift die Klinke 118 in den Abschnitt mit den Rastzähnen 42 ein, deren Form aus Fig. 14 hervorgeht und bereits beschrieben wurde. Bei der Verdrehung schnappt die Klinke 118 hinter jedem Zahn 42 ein und erzeugt dabei ein Klickgeräusch, das vom Benutzer gezählt werden kann und ihm so eine akustische Information über die eingestellte Dosis gibt, wenn er blind oder sehschwach ist. Fig. 14 zeigt rein beispielhaft eine Verdrehung um 54° in Richtung des dortigen Pfeiles 128, entsprechend drei Rastzähnen 42. (Pro 180° sind beim Ausführungsbeispiel zehn Rastzähne 42 vorgesehen, d.h. der Winkelabstand α zwischen zwei Rastzähnen 42 beträgt beim Ausführungsbeispiel 18°).

Genau um diese 54° wird dabei nicht nur die Verstellhülse 15 verdreht, sondern ebenso das sich in dieser Stellung frei bewegende Führungsglied 67 und ebenso die Gewindespindel 19, d.h. an der Gesamtlänge des Stößels 80 (Fig. 3) ändert sich durch diesen Verstellvorgang absolut nichts. Der Stößel 80 ist nicht länger geworden. Wie soll da etwas injiziert werden?

Zum Auslösen des Injektionsvorgangs dient nun der federnde Clipabschnitt 109, der mit einem nach innen ragenden Vorsprung 130 versehen ist, welcher der Öffnung 104 gegenüberliegt und in diese eintauchen kann, wenn man mit einem Finger in Richtung des Pfeiles 131 (Fig. 10) auf diesen Clipabschnitt 109 drückt.

Hierbei wird der Rastzapfen 62 nach innen gedrückt und hält nun nicht mehr den Stößel 80 (Fig. 3) in dessen gespannter Lage fest, so daß dieser durch die gespannte Feder 53 in proximaler Richtung verschoben wird. Wir werden nachfolgend sehen, daß durch diese Verschiebung die Injektionsnadel 74 eingestochen wird, wie das Fig. 19 zeigt.

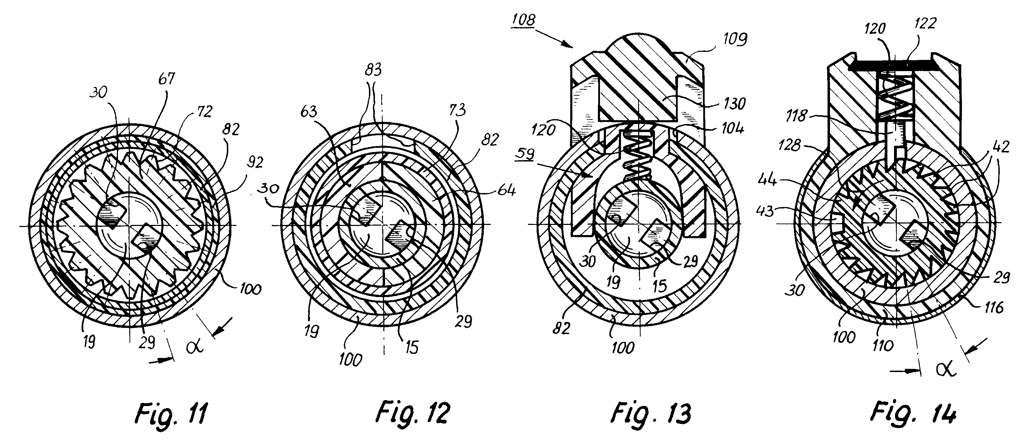

Durch diese Verschiebung gleitet nun das Führungsglied 67 mit seiner Außenverzahnung 72 in die Innenverzahnung 93. Ein Blick auf Fig. 11 belehrt uns, daß diese Verzahnungen dieselbe Zahnteilung (beim Ausführungsbeispiel: 18°) haben, wie die Rastzähne 42, so daß also in jeder der möglichen Raststellungen der Verstellhülse 15 die Außenverzahnung 72 leicht und ohne Probleme in die Innenverzahnung 93 hineingleiten kann. (Das Teil 92 wird, wie beschrieben, in einer entsprechenden, vorgegebenen Drehstellung an der Hülse 82 befestigt.)

Während also die Außenverzahnung 72 sanft in die Innenverzahnung 93 hineingleitet und dadurch das Führungsglied 67 sicher an jeder Drehung relativ zum Gehäuse (Rohr 100) des Teils A gehindert wird, gleitet die Klinke 118 aus dem Bereich mit den Rastzähnen 42 in den Bereich 40' der Verstellhülse 15. Dabei ergibt sich eine Überlappung, d.h. erst, wenn die Außenverzahnung 72 im Eingriff mit der Innenverzahnung 93 ist, gleitet die Klinke 118 voll aus den Rastzähnen 42 heraus.

Hat die Klinke 118 die Rastzähne 42 voll verlassen, so kann sie eine Verdrehung der Verstellhülse 15 durch die auf Torsion gespannte Feder 53 nicht mehr verhindern, und diese Feder 53 verdreht nun die Verstellhülse 15 um den zuvor eingestellten Winkel wieder zurück, d.h. wenn man auf das distale Ende des Betätigungsknopfs 38 schaut, wird dieser, wenn er die Stellung nach Fig. 7 wieder erreicht hat, um den zuvor (bei der Dosiseinstellung eingestellten) Winkel entgegen dem Uhrzeigersinn wieder zurückgedreht. Beim Beispiel gemäß Fig. 14 mit drei Rastzähnen 42 entsprechend 54° wird also die Verstellhülse 15 um 54° entgegen dem Uhrzeigersinn zurückgedreht. Da hierbei das Führungsglied 67 relativ zum Gehäuse (Rohr 100) unverdrehbar ist, bewirkt erst diese Verdrehung der Verstellhülse 15 ein Herausschrauben der Gewindespindel 19 aus dem Innengewinde 17 der Verstellhülse 15, und damit die Injektion der eingestellten Dosis.

Man erkennt nun auch die großen Vorteile:
a) Bis kurz vor Ende des Injektionsvorgangs behält der Stößel 80 (Fig. 3) seine frühere Länge, und erst am Ende des Injektionsvorgangs wird er länger. Das ermöglicht, wie man ohne weiteres einsieht, eine sehr kurze Bauweise des Injektors.
b) die Injektion der eingestellten Dosis erfolgt erst, wenn die Nadel bereits im Patienten eingestochen ist.
c) Auch wenn sich etwas Luft in der Kartusche 11 befindet, geht keine Injektionsflüssigkeit verloren. Luft in der Kartusche 11 wirkt nämlich dort wie eine Feder und verlangsamt den Injektionsvorgang. Der Patient muß lediglich die Nadel 74 so lange eingestochen lassen, bis der Injektionsvorgang abgeschlossen ist. Dies wird ihm beim Training mit diesem Injektor beigebracht.
d) Nach der Injektion befindet sich der Injektor 10 in seiner Nullstellung, d.h. der Wert "0" der Skala 46 (Fig. 6) liegt automatisch, und ohne daß der Patient etwas dazu tun muß, wieder der Gehäusemarke 47 gegenüber, die sich auf der Blende 116 befindet.
e) Folglich reduziert sich eine Injektion auf folgende einfachen Schritte:
   Spannen
   Dosis einstellen
   Gerät ansetzen
   Auslösen.

Bei Verwendung in der Veterinärmedizin ergibt sich so eine große Zeitersparnis, und bei Verwendung in der Humanmedizin werden Fehlbedienungen ganz weitgehend ausgeschlossen, da der Patient eigentlich nichts mehr falsch machen kann. Er kann allenfalls vergessen, seine Injektionsdosis einzustellen, wobei in diesem Fall überhaupt nichts injiziert wird. Dies ist unvermeidbar, denn das Gerät kann ja nicht die Dosis "erraten", die der Patient im Augenblick braucht und die er oft erst kurz zuvor selbst bestimmt hat. Ggf. kann man aber in der Stellung "0" eine Auslösung des Injektors 10 blockieren, wie das z.B. in der EP 0 349 592 B1 beschrieben ist, wodurch der Patient daran erinnert wird, daß er vor dem Auslösen die Injektionsdosis einstellen muß.

Die Möglichkeit, auch in der Dosisstellung "0" eine Injektion auszulösen, hat den Vorteil, daß man das Gerät zur Blutentnahme verwenden kann, wenn man eine Spezialnadel verwendet, wie sie in der DE 38 42 317 A1 dargestellt und beschrieben ist. Bei dieser Nadel wird, wie üblich, eine sterile Abdeckung für die Nadel verwendet, aber diese Abdeckung trägt an ihrem proximalen Ende eine kleine Lanzette für die Blutentnahme. In der Dosisstellung "0" kann man nun das erfindungsgemäße Gerät dazu verwenden, diese Lanzette schmerzfrei in den Patienten einzustechen. Dies ist deshalb möglich, weil bei der Dosiseinstellung "0" das Gerät kein Auspressen von Injektionsflüssigkeit bewirkt und die Nadel 74, bzw. die Lanzette, hierbei durch den Teil 73 des Führungsgliedes 67 angetrieben wird und nicht durch die Gewindespindel 19 wie bei der vorstehend erwähnten EP 0 349 592 B1. Dieser "Direktantrieb" der Nadel, wie er nachfolgend noch ausführlich beschrieben wird, erweist sich in der Praxis als sehr vorteilhaft und bringt auch deshalb mehr Sicherheit, weil er ausschließt, daß sich der Patient selbst dann Injektionsflüssigkeit injiziert, wenn das Gerät auf die Dosis "0" eingestellt ist.

### Der B-Teil (Fig. 15 und 16)

Fig. 15 zeigt die verschiedenen Einzelteile des B-Teils, und Fig. 16 den B-Teil im zusammengebauten Zustand, im Längsschnitt.

Zur Verbindung mit dem A-Teil dient das Außengewinde 13 an einem rohrartigen Teil 135, welch letzteres z.B. aus Aluminium hergestellt sein kann. Das Teil 135 hat, beginnend beim Außengewinde 13, zunächst einen zylindrischen Abschnitt 136, der bevorzugt denselben Durchmesser hat wie das Rohr 100. Über eine Schulter 137 geht der Abschnitt 136 über in einen zylindrischen Abschnitt 138 etwas kleineren Durchmessers, und dieser geht seinerseits über eine Schulter 139 über in einen Abschnitt 140 kleineren Durchmessers, dessen proximaler Bereich mit einem Außengewinde 143 versehen ist.

Der Abschnitt 140 hat, einander gegenüberliegend, zwei Langlöcher 144, 145, die bei der dargestellten Ausführungsform aus fertigungstechnischen Gründen in axialer Richtung etwas gegeneinander versetzt sind, d.h. das Langloch 145 liegt näher beim proximalen Ende des rohrartigen Teils als das Langloch 144, wie aus Fig. 16 klar hervorgeht.

Ferner hat der Abschnitt 140 zwei diametral gegenüberliegende Sichtfenster 27, die bereits beschrieben wurden und die dazu dienen, den Füllstand der Kartusche 11 zu beobachten. (Diese ist in Fig. 15 nicht dargestellt.) Die zylindrische Innenausnehmung des Abschnitts 140 ist mit 141 (Fig. 16) bezeichnet.

Ein Kartuschenhalter 148 ist zur Aufnahme im rohrartigen Teil 135 ausgebildet und dient seinerseits, wie in Fig. 16 dargestellt, zur Aufnahme einer Kartusche 11. Er wird gewöhnlich aus Metall ausgebildet, z.B. aus Aluminium, kann aber auch aus einem stabilen Kunststoff hergestellt werden.

Der Kartuschenhalter 148, der im rohrartigen Teil 135 verschiebbar angeordnet ist, verjüngt sich an seinem proximalen Ende und bildet dort einen radial nach innen ragenden Flansch 149, gegen den das proximale Ende 150 einer eingesetzten Kartusche 11 anliegt. Das proximale Ende des Kartuschenhalters 148 ist mit einem Außengewinde 152 versehen, auf das eine Hülse 153 aufgeschraubt werden kann, in der die Nadel 74 befestigt ist. Die Kartusche 11 hat in der üblichen Weise an ihrem proximalen Ende eine - nicht dargestellte - dünne Gummimembran, die vom distalen Ende der Nadel 74 durchstochen wird. Die Nadel 74 kann so nach einer Injektion leicht getauscht werden, wie das dem Fachmann bekannt ist.

Der Kartuschenhalter 148 hat ferner einen im wesentlichen zylindrischen Außenabschnitt 155, der sich in distaler Richtung an das Außengewinde 152 anschließt und sich bis zu einer Ringschulter 156 am distalen Ende des Kartuschenhalters 148 erstreckt, welche Ringschulter 156 zur Anlage einer Schraubenfeder 157 dient, die im montierten Zustand um den Kartuschenhalter 148 herum angeordnet ist und deren anderes Ende im montierten Zustand gegen das distale Ende der Ringschulter 139 anliegt.

Die zylindrische Innenausnehmung des Kartuschenhalters 148 ist mit 158 bezeichnet. An ihrem distalen Ende ist sie mit einem Innengewinde 160 versehen, in das im montierten Zustand eine außen gerändelte Schraube 162 mit einem entsprechenden Außengewinde 163 eingeschraubt ist, welche die Kartusche 11 in der Innenausnehmung 158 festhält, vgl. Fig. 16. Die Rändelschraube 162 hat eine zentrale Öffnung 161, die etwa denselben Durchmesser hat wie das Innere der Kartusche 11. Durch diese Öffnung 161 kann, wie in Fig. 16 dargestellt, die Gewindespindel 19 ungehindert durchtreten. Andererseits ist, wie z.B. Fig. 19 zeigt, die Schraube 162 so ausgelegt, daß zu Beginn einer Injektion dann, wenn die Feder 53 den Abschnitt 73 des Führungsglieds 67 in proximaler Richtung bewegt, dieser Abschnitt 73 gegen das distale Ende der Schraube 162 anliegt und diese, in Richtung der Pfeile 164 der Fig. 19, entgegen der Wirkung der Feder 157 in proximaler Richtung verschiebt und so die Nadel 74 in den Patienten einsticht, und dies, bevor die Gewindespindel 19 tätig wird und die zuvor eingestellte Flüssigkeitsmenge aus der Kartusche 11 auspreßt.

Die Feder 157 wird also auf den Kartuschenhalter 148 aufgeschoben, und dieser wird dann in die zylindrische Innenausnehmung 141 des rohrartigen Teils 135 hineingeschoben.

Der Kartuschenhalter 148 hat auf seiner Außenseite zwei radial nach außen ragende Vorsprünge 168, 169, die jeweils auf einer federnden Zunge 170 bzw. 171 angeordnet sind. Diese Vorsprünge 168, 169 sind um das gleiche Maß gegeneinander versetzt wie die beiden Langlöcher 144, 145, und sie werden bei der Montage etwas nach innen gedrückt und schnappen dann nach der Montage jeder in "sein" Langloch, also der Vorsprung 168 in das Langloch 144, und der Vorsprung 169 in das Langloch 145. Sie begrenzen so die axiale Verschiebung des Kolbenhalters 148 im rohrartigen Teil 135 in beiden Richtungen.

Beim Einstechen der Nadel 74 in den Patienten werden die Vorsprünge 168, 169 bis zum proximalen Ende der zugeordneten Langlöcher 144 bzw. 145 verschoben und begrenzen dadurch die Einstichtiefe der Nadel 74. Beim Einsetzen einer neuen Kartusche 11 begrenzen sie die Bewegung des Kartuschenhalters 148 in distaler Richtung, also aus dem rohrartigen Teil 135 heraus, wobei es dann gut möglich ist, den gerändelten Teil der Schraube 162 mit den Fingern zu ergreifen und abzuschrauben, z.B. zur Entnahme der Kartusche 11 oder zum Einsetzen einer neuen. (Diese Stellung des Kartuschenhalters 148 ist in Fig. 16 nicht dargestellt).

Der Kartuschenhalter 148 hat zwei Sichtlöcher 175 (Fig. 16) und 176 (Fig. 15) in Form von Langlöchern, welche den Sichtöffnungen 27 des rohrartigen Teils 135 gegenüberliegen und es ermöglichen, den Füllungszustand der Kartusche 11 ständig zu überwachen, ebenso die Lage der Gewindespindel 19, wie eingangs beschrieben. Da sich der Kartuschenhalter 148 bei Benutzung im Teil 135 verschiebt, müssen seine Sichtlöcher 175, 176 länger sein als die Sichtöffnungen 27, wie man ohne weiteres einsieht.

Nach dem Einsetzen des Kartuschenhalters 148 in das rohrartige Teil 135 kann nun ein Rohrstück 174 montiert werden, das ebenfalls mit zwei Sichtlöchern 177, 177' (Fig. 15) versehen ist, die sich diametral gegenüberliegen. Diese Sichtlöcher 177, 177' werden von innen mittels eines Clip 178 aus transparentem Kunststoff verschlossen, der zwei kleine Scheiben 179 (Fig. 15) trägt, welche nach der Montage genau in die Sichtlöcher 177, 177' passen und diese staubdicht verschließen, so daß keine Fremdkörper und kein Schmutz in den Injektor 10 eindringen und dessen Funktion behindern können.

Das mit dem Clip 178 versehene Rohrstück 174 wird auf den zylindrischen Abschnitt 138 des rohrartigen Teils 135 aufgeschoben und dort gesichert, z.B. durch Kleben. Es hat denselben Außendurchmesser wie das Rohr 100.

Anschließend wird eine Hülse 182 für die Stichtiefeneinstellung, die z.B. aus Aluminium besteht und an ihrem distalen Ende mit einem Innengewinde 183 versehen ist, mittels dieses Innengewindes 183 auf das Außengewinde 143 des rohrartigen Teils 135 aufgeschraubt. Das Innengewinde 183 befindet sich an einem Abschnitt 184 reduzierten Durchmessers der Hülse 182, und dieser Abschnitt 182 wird bei der Montage unter das proximale Ende des Rohres 174 geschraubt. Durch Verdrehen des Rohres 182 kann die Einstichtiefe der Nadel 74 vom Benutzer verändert werden, d.h. wenn die Hülse 182 in proximaler Richtung herausgeschraubt wird, sticht die Nadel 74 weniger tief ein.

### Arbeitsweise des Injektors 10

Zum Laden mit einer neuen Kartusche 11 werden A-Teil und B-Teil an der Schraubverbindung 13 auseinandergeschraubt. Die Rändelschraube 162 wird vom Kartuschenhalter 148 abgeschraubt, die alte Kartusche entnommen und eine neue Kartusche 11 wird eingesetzt, und die Rändelschraube 162 wird dann wieder eingeschraubt.

A-Teil und B-Teil bleiben zunächst getrennt, und der A-Teil wird jetzt in die gespannte Stellung gemäß Fig. 10 gebracht, und in dieser Stellung wird die Gewindespindel 19 durch Drehen von Hand ganz in das Führungsglied 15 eingeschraubt, also noch mehr, als das in Fig. 10 dargestellt ist. Anschließend wird der A-Teil durch Druck auf den Clip 109 ausgelöst, wodurch er die Stellung nach Fig. 7 einnimmt.

Nunmehr können A-Teil und B-Teil wieder (mittels der Gewinde 13) zusammengeschraubt werden, wodurch sich das Bild nach Fig. 17 ergibt, d.h. das Gerät befindet sich in seiner Grundstellung.

Dabei drückt das Teil 73 des Führungsglieds 67 auf die Rändelschraube 162 und drückt dadurch die Feder 157 etwas zusammen.

Der Benutzer macht nun zweckmäßig eine Probeinjektion, d.h. er bringt den Injektor 10 in die Spannstellung gemäß Fig. 18, wodurch jetzt die Rändelschraube 162 durch die Feder 157 in Anlage gegen das Teil 92 des A-Teils gebracht wird.

Nach dem Spannen verdreht der Benutzer den Betätigungsknopf 38 zur Einstellung einer Dosis. Dies ist in Fig. 18 bereits geschehen und deshalb durch einen gestrichelten Drehpfeil 190 angedeutet. Dieser Vorgang der Dosiseinstellung wurde schon weiter oben bei der Beschreibung der Arbeitsweise des A-Teils ausführlich beschrieben.

Gemäß Fig. 19 wird dann ausgelöst, wodurch sich das Teil 73 des Führungsglieds 67 in proximaler Richtung bewegt und auf die Rändelschraube 162 drückt, was in Fig. 19 durch die Pfeile 164 angedeutet ist. Auch dies wurde bereits weiter oben bereits ausführlich beschrieben. Hierbei wird die Feder 157, die schwächer ist als die Feder 53, zusammengepreßt, und die Nadel 74 kommt aus der Hülse 182 hervor. Zweckmäßig wird bei dieser Probe die Nadel 74 so gehalten, daß sie nach oben zeigt, damit bei diesem Vorgang etwa vorhandene Luftblasen aus der Kartusche 11 sicher entfernt werden.

Es hat sich gezeigt, daß das Verhältnis der Kräfte der Federn 53 und 157 für einen optimalen Ablauf dieses Vorgangs wichtig ist, und zwar darf die Feder 157 nicht zu schwach sein. Da nämlich die Feder 53 auch die Funktion hat, ein Drehmoment zu erzeugen, muß sie hauptsächlich hierfür ausgelegt werden und ist daher - bezogen auf ihre Fähigkeit, eine axiale Kraft zu erzeugen - recht stark. Deshalb muß ihr die Feder 157 entgegenwirken, damit der Vorgang des Einstechens der Nadel 74 nicht zu schnell abläuft, was zu Hämatomen führen könnte, und damit das Spannen des Injektors 10 besonders für ältere Patienten nicht zu schwierig wird. Insofern spielt auch die Differenz der Kräfte beider Federn - die ja beim Spannvorgang einander entgegenwirken - eine wichtige Rolle.

Bei einer praktischen Ausführungsform hat die Feder 53 im gespannten Zustand, also mit der Länge gemäß Fig. 10, eine Kraft von 18 N, und die Feder 157 hat im gespannten Zustand, also mit der Länge gemäß Fig. 17, eine Kraft von 6 N, d.h. das Verhältnis der Kräfte - jeweils im komprimierten Zustand - beträgt hier 3:1. Genaue Werte lassen sich im Einzelfall nur empirisch bestimmen, da beide Federn sowohl auf ihrer Außenseite wie auf ihrer Innenseite an Teilen des Injektors reiben, und diese Friktionseinflüsse sind nicht quantifizierbar und müssen empirisch, also durch trial and error, ermittelt werden. Die Werte 6 N und 18 N, die als Hinweise auf Größenordnungen zu verstehen sind, beziehen sich auf Federn, die - ohne Reibung - in einem Meßgerät auf entsprechende Längen zusammengedrückt sind und dort gemessen werden. Nach Einbau im Injektor sind, bedingt durch die Reibung, die gemessenen Werte kleiner.

Selbstverständlich ist es im Rahmen der Erfindung nicht ausgeschlossen, die Doppelfunktion der Feder 53, also das Erzeugen einer axialen Kraft für das Einstechen der Nadel, und das Erzeugen eines Drehmoments für den Vorgang der Injektion der zu injizierenden Flüssigkeitsmenge, auf zwei verschiedene Federn zu verteilen, von denen dann jede nur eine einzige Funktion hat. Die dargestellte Ausführungsform hat aber den Vorzug größerer Einfachheit, da man für beide Funktionen nur eine einzige Feder 53 benötigt.

Befindet sich bei der Probeinjektion die Nadel 74, wie in Fig. 19 dargestellt, in ihrer Injektionsstellung, in der sie normalerweise in den Patienten eingestochen wäre, so erfolgt der eigentliche Vorgang des Auspressens von Flüssigkeit (und ggf. Luft) aus der Kartusche 11, wie er in Fig. 20 dargestellt ist. Auch dieser Vorgang wurde bereits weiter oben bei der Beschreibung der Arbeitsweise des A-Teils ausführlich beschrieben. Hierbei dreht sich der Betätigungsknopf 38, und mit ihm die Verstellhülse 15, in Richtung der Pfeile 191, 192 der Fig. 20, während die Außenverzahnung 72 des Führungsglieds 67 im Teil 92 gegen Verdrehung festgehalten wird. Dadurch bewegt sich die Gewindespindel 19 in Richtung des Pfeiles 193 in proximaler Richtung und verschiebt den Kolben 23 in proximaler Richtung in der Kartusche 11, die ihrerseits nicht mehr im Gehäuse des Injektors 10 in proximaler Richtung verschoben werden kann, da sich die Zapfen 168, 169 des Kartuschenhalters 148 bereits im Anschlag gegen die proximalen Enden der Langlöcher 144, 145 befinden. Hierdurch wird eine Quantität Injektionsflüssigkeit 194 gemäß der zuvor eingestellten Dosis aus der Nadel 74 ejiziert. Der Benutzer sieht das und weiß, daß der Injektor 10 nun gebrauchsfertig ist und etwaige Luft aus ihm entfernt worden ist.

Der Benutzer kann dann den eben beschriebenen Ablauf entsprechend den Fig. 17 bis 20 erneut durchführen und dabei sich selbst die erforderliche Dosis injizieren.

Naturgemäß sind im Rahmen der vorliegenden Erfindung außerordentlich viele Abwandlungen und Modifikationen möglich, wie das dem Fachmann ohne weiteres ersichtlich ist. Z.B. könnten die Gehäuseteile statt aus Aluminium auch aus einem geeigneten Kunststoff hergestellt werden, während umgekehrt stark beanspruchte Teile im Inneren des Injektors 10 aus Metall hergestellt werden können. In vielen Fällen wird es z.B. zweckmäßig sein, den Durchmesser des Teils 75 etwas kleiner zu wählen als den Durchmesser der Schulter 48, damit keine Gefahr besteht, daß das Teil 75 beim Dosiervorgang irgendwo streift. Insgesamt bietet der erfindungsgemäße Injektor speziell für die sogenannte intensivierte Insulintherapie außerordentlich viele Vorteile, da sich ein Benutzer aus einer einzigen Kartusche 11, enthaltend z.B. 100 IE Insulin, je nach Bedarf zwanzig oder mehr Injektionen geben kann, bevor eine neue Kartusche eingesetzt werden muß, wobei der Ablauf der Injektionen - wie beschrieben - außerordentlich einfach und narrensicher ist. Es bedarf auch keiner Erwähnung, daß sich der erfindungsgemäße Injektor außer für Insulin auch für beliebige andere Flüssigkeiten eignet, die injiziert werden müssen, z.B. für Vitamin B₁₂ bei der Behandlung der Anämie.

## Patentansprüche

1. Injektor zur Aufnahme einer Kartusche (11) mit einer gewöhnlich für eine Mehrzahl von Injektionen ausreichenden Menge an Injektionsflüssigkeit (12),
welche Kartusche (11) im Injektor (10) gegen die Kraft einer Rückstellfeder (157) in proximaler Richtung verschiebbar ist, mit einem durch eine Feder (53) in proximaler Richtung beaufschlagten, längenverstellbaren Stößel (Fig. 3: 80), welcher im Injektor (10) zwischen einer proximalen Endstellung (Fig. 7) und einer distalen Endstellung (Fig. 10) verschiebbar ist und eine Gewindespindel (19) aufweist, die im Gewinde (17) eines Verstellglieds (15) geführt ist, zur Beaufschlagung eines in der Kartusche (11) vorgesehenen Kolbens (23) dient, und der ein Führungsglied (67) zugeordnet ist, das mit ihr drehfest, aber axial verschiebbar, verbunden ist,
dadurch gekennzeichnet, daß das Führungsglied (67) in der distalen Endstellung (Fig. 10) des Stößels (80) relativ zum Gehäuse (100) des Injektors (10) verdrehbar ist, in der proximalen Endstellung (Fig. 7) aber nicht.

2. Injektor nach Anspruch 1, dadurch gekennzeichnet, daß zwischen dem Gehäuse (100) und dem Verstellglied (15) eine Feder (53) vorgesehen ist, deren Vorspannung durch Verdrehen des Verstellglieds (15) veränderbar ist,
und daß in der distalen Endstellung (Fig. 10) des Stößels (80) eine Sperrvorrichtung (42, 118) vorgesehen ist, welche ein solches Verdrehen des Verstellglieds (15) nur in einer bestimmten Richtung ermöglicht und eine Drehung in der umgekehrten Richtung sperrt.

3. Injektor nach Anspruch 1 und 2, dadurch gekennzeichnet, daß die Feder (53), deren Vorspannung durch Verdrehen des Verstellgliedes (15) veränderbar ist, auch zum Beaufschlagen des längenverstellbaren Stößels (Fig. 3: 80) in proximaler Richtung dient.

4. Injektor nach Anspruch 3, dadurch gekennzeichnet, daß die zum Beaufschlagen des längenverstellbaren Stößels (Fig. 3: 80) dienende Feder (53) beim Injektionsvorgang zuerst Energie freigibt, die in ihr durch axiale Kompression gespeichert wurde, um eine axiale Kraft für das Einstechen der Nadel (74) des Injektors zu bewirken,
und anschließend Energie freigibt, welche in dieser Feder durch Verdrehung gespeichert wurde, um ein Drehmoment für die Injektion der zu injizierenden Flüssigkeitsmenge zu erzeugen.

5. Injektor nach einem oder mehreren der Ansprüche 2 bis 4, dadurch gekennzeichnet, daß die Sperrvorrichtung (42, 118) in der proximalen Endstellung (Fig. 10) des Stößels (80) nicht wirksam ist.

6. Injektor nach einem oder mehreren der Ansprüche 2 bis 4, und nach Anspruch 5, dadurch gekennzeichnet, daß das Verstellglied (15) in der distalen Endstellung (Fig. 10) des Stößels (80) ausgehend von einer vorgegebenen Drehstellung (Anschlag 44) durch Verdrehen verstellbar ist, und daß die Verdrehung des Verstellglieds (15) in der proximalen Endstellung (Fig. 7), die dort in umgekehrter Richtung abläuft, bis zu dieser vorgegebenen Drehstellung (Anschlag 44) begrenzt ist.

7. Injektor nach einem oder mehreren der Ansprüche 2 bis 6, dadurch gekennzeichnet, daß die Sperrvorrichtung eine am Gehäuse (100) angeordnete Sperrklinke (118) und mit dem Verstellglied (15) verbundene Ratschenzähne (42), oder umgekehrt, aufweist, wobei die Sperrklinke (118) in der distalen Endstellung (Fig. 10) des Stößels (80) in diese Ratschenzähne (42) eingreift.

8. Injektor nach einem oder mehreren der vorhergehenden Ansprüche, dadurch gekennzeichnet, daß das Führungsglied (67) mit dem Verstellglied (15) drehbar, aber axial unverschieblich, verbunden ist.

9. Injektor nach Anspruch 8, dadurch gekennzeichnet, daß zwischen dem Führungsglied (67) und dem Verstellglied (15) mindestens ein elastisch verformbares Element (63, 64) vorgesehen ist, welches mit Vorspannung zwischen dem Führungsglied (67) und dem Verstellglied (15) angeordnet und mit mindestens einem dieser beiden Glieder (15, 67) formschlüssig verbunden ist.

10. Injektor nach Anspruch 8 oder 9, dadurch gekennzeichnet, daß das Führungsglied (67) mit wenigstens einem Eingriffselement (72) versehen ist, welches in der proximalen Endstellung (Fig. 7) des Stößels (Fig. 3: 80) mit wenigstens einem hierzu im wesentlichen komplementären gehäusefesten Eingriffselement (93) des Injektors (10) in Eingriff steht.

11. Injektor nach Anspruch 10, dadurch gekennzeichnet, daß das Eingriffselement des Führungsglieds (67) als Längsverzahnung (72) ausgebildet ist, und daß dieser Längsverzahnung (72) eine entsprechende gehäusefeste Längsverzahnung (93) zugeordnet ist, welche mit der Längsverzahnung (72) des Führungsglieds (67) mindestens in der proximalen Endstellung (Fig. 7) des Stößels (Fig. 3: 80) in Eingriff steht.

12. Injektor nach den Ansprüchen 7 und 11, dadurch gekennzeichnet, daß die Ratschenzähne (42) und die Längsverzahnung (72) eine miteinander kompatible Zahnteilung (Fig. 11 und 14: Winkel alpha) aufweisen.

13. Injektor nach Anspruch 12, dadurch gekennzeichnet, daß die Zahnteilung der Ratschenzähne (42) und die Zahnteilung der Längsverzahnung (72) Werte aufweisen, bei denen der größere durch den kleineren ganzzahlig teilbar ist.

14. Injektor nach Anspruch 12 oder 13, dadurch gekennzeichnet, daß die Zähne der Längsverzahnung (72) so relativ zu der den Ratschenzähnen (42) zugeordneten Sperrklinke (118) angeordnet sind, daß bei einer Bewegung des Stößels (Fig. 3: 80) von dessen distaler Endstellung (Fig. 10) in dessen proximale Endstellung (Fig. 7) eine im wesentlichen verdrehungsfreie Linearbewegung des Stößels (80) und des auf ihm angeordneten Führungsglieds (67) möglich ist.

15. Injektor nach einem oder mehreren der vorhergehenden Ansprüche, dadurch gekennzeichnet, daß die Gewindespindel (19) aus Kunststoff ausgebildet ist und symmetrisch zueinander angeordnete Längsnuten (29, 30) zum Eingriff mit einem Führungselement (69) des Führungsglieds (67) aufweist.

16. Injektor nach einem oder mehreren der vorhergehenden Ansprüche, dadurch gekennzeichnet, daß die Gewindespindel (19) in Längsrichtung in zwei visuell unterscheidbare Bereiche (24, 25) unterteilt ist.

17. Injektor nach Anspruch 16, dadurch gekennzeichnet, daß ein Bereich (25) der Gewindespindel (29) farbig ausgebildet ist.

18. Injektor nach einem oder mehreren der vorhergehenden Ansprüche, dadurch gekennzeichnet, daß das Führungsglied (67) dazu ausgebildet ist, beim Injektionsvorgang die Kartusche (11) oder einen diese aufnehmenden Kartuschenträger (148, 162) durch direkte Anlage gegen diese bzw. diesen in proximaler Richtung zu verschieben (Fig. 19: Pfeile 164).

19. Injektor nach einem oder mehreren der Ansprüche 2 bis 18, dadurch gekennzeichnet, daß die Sperrvorrichtung (42, 118) so ausgebildet ist, daß sie bei der Bewegung des Stößels (Fig. 3: 80) von dessen distaler Endstellung (Fig. 10) zu dessen proximaler Endstellung (Fig. 7) mindestens so lange die Drehung des Verstellglieds (15) in der genannten umgekehrten Drehrichtung sperrt, wie das Führungsglied (67) relativ zum Gehäuse (100) frei verdrehbar ist.

20. Injektor nach einem oder mehreren der vorhergehenden Ansprüche, dadurch gekennzeichnet, daß das Verstellglied (15) mit einem außerhalb des Gehäuses (100) angeordneten Betätigungsorgan (38) verbunden ist, welches die Verstellung des Stößels (Fig. 3: 80) von dessen proximaler Endstellung (Fig. 7) in dessen distale Endstellung (Fig. 10) unter gleichzeitiger Spannung der dem Stößel (80) zugeordneten Feder (53) ermöglicht, und welches in der distalen Endstellung (Fig. 10) eine Verdrehung des Betätigungsorgans (38) zur Vorwahl einer gewünschten Injektionsdosis (Fig. 6: 46) ermöglicht.

21. Injektor nach Anspruch 20, dadurch gekennzeichnet, daß dem Stößel (Fig. 3: 80) ein lösbares Rastorgan (59, 104) zum Einrasten in dessen distaler Endstellung (Fig. 10) zugeordnet ist.

22. Injektor nach einem oder mehreren der vorhergehenden Ansprüche, dadurch gekennzeichnet, daß dem Stellglied (38) eine Skala (Fig. 6: 46) zum Ablesen der eingestellten Dosis zugeordnet ist, welche Skala bei der beim Auspressen der zuvor eingestellten Injektionsdosis auftretenden Bewegung wieder auf Null gestellt wird.

23. Injektor zur Aufnahme einer Kartusche (11) mit einer gewöhnlich für eine Mehrzahl von Injektionen ausreichenden Menge an Injektionsflüssigkeit (12), welche Kartusche (11) im Injektor (10) gegen die Kraft einer Rückstellfeder (157) in proximaler Richtung verschiebbar ist,
mit einem durch eine Feder (53) in proximaler Richtung beaufschlagten längenverstellbaren Stößel (Fig. 3: 80), welcher im Injektor (10) zwischen einer proximalen Endstellung (Fig. 7) und einer distalen Endstellung (Fig. 10) verschiebbar ist und eine Gewindespindel (19) aufweist, die im Gewinde (17) eines Verstellglieds (15) geführt ist, zur Beaufschlagung eines in der Kartusche (11) vorgesehenen Kolbens (23) dient, und der ein Führungsglied (67) zugeordnet ist, das mit ihr drehfest, aber axial verschieblich, verbunden ist, dadurch gekennzeichnet, daß das Führungsglied (67) dazu ausgebildet ist, bei der beim Injektionsvorgang erfolgenden proximalen Verschiebung der Kartusche (11) diese, oder einen sie aufnehmenden Kartuschenträger (148, 162), direkt zu beaufschlagen (Fig. 19: Pfeile 164) und zu verschieben,
und daß das Führungsglied (67) in der distalen Endstellung (Fig. 10) des Stößels (80) relativ zum Gehäuse (100) des Injektors (10) verdrehbar ist, in der proximalen Endstellung (Fig. 7) aber nicht.

24. Injektor nach Anspruch 23, dadurch gekennzeichnet, daß zwischen dem Gehäuse (100) und dem Verstellglied (15) eine Feder (53) vorgesehen ist, deren Vorspannung durch Verdrehen des Verstellglieds (15) veränderbar ist,
und daß in der distalen Endstellung (Fig. 10) des Stößels (80) eine Sperrvorrichtung (42, 118) vorgesehen ist, welche ein solches Verdrehen des Verstellglieds (15) nur in einer bestimmten Richtung ermöglicht und eine Drehung in der umgekehrten Richtung sperrt.

25. Injektor nach Anspruch 23 und 24, dadurch gekennzeichnet, daß die Feder (53), deren Vorspannung durch Verdrehen des Verstellgliedes (15) veränderbar ist, auch zum Beaufschlagen des längenverstellbaren Stößels (Fig. 3: 80) in proximaler Richtung dient.

26. Injektor nach Anspruch 25, dadurch gekennzeichnet, daß die zum Beaufschlagen des längenverstellbaren Stößels (Fig. 3: 80) dienende Feder (53) beim Injektionsvorgang zuerst Energie freigibt, die in ihr durch axiale Kompression gespeichert wurde, um eine axiale Kraft für das Einstechen der Nadel (74) des Injektors zu bewirken,
und anschließend Energie freigibt, welche in dieser Feder durch Verdrehung gespeichert wurde, um ein Drehmoment für die Injektion der zu injizierenden Flüssigkeitsmenge zu erzeugen.

27. Injektor nach einem oder mehreren der vorhergehenden Ansprüche, dadurch gekennzeichnet, daß die Sperrvorrichtung (42, 118) in der proximalen Endstellung (Fig. 7) des Stößels (80) nicht wirksam ist.

## Claims

1. Injection device for holding a cartridge (11) containing a quantity of injection fluid (12) typically adequate for multiple injections, said cartridge (11) being displaceable in a proximal direction in the injection device (10) counter to the force of a resetting spring (157), comprising an adjustable-length tappet (Fig. 3: 80) acted upon in the proximal direction by a spring (53), said tappet being displaceable in the injection device (10) between a proximal end position (Fig. 7) and a distal end position (Fig. 10) and having a threaded spindle (19), guided in the thread (17) of an adjusting member (15) for action upon a plunger (23) provided in the cartridge (11), a guide member (67) being associated with said threaded spindle and coupled thereto in a manner fixed against relative rotation but axially displaceably, characterised in that the guide member (67) is rotatable relative to the housing (100) of the injection device (10) in the distal and position (Fig. 10) of the tappet (80), but not in the proximal end position (Fig. 7) thereof.

2. Injection device according to claim 1, characterised in that a spring (53) is provided between the housing (100) and the adjusting member (15), whose biasing is variable by rotation of the adjusting member (15), and in that in the distal end position (Fig. 10) of the tappet (80) a blocking device (42, 118) is provided which enables such rotation of the adjusting member (15) only in a predetermined direction and blocks a rotation in the opposite direction.

3. Injection device according to claim 1 and 2, characterised in that the spring (53), whose biasing is variable by rotation of the adjusting member (15), also serves for action upon the adjustable-length tappet (Fig. 3: 80) in the proximal direction.

4. Injection device according to claim 3, characterised in that during the injection process the spring (53) serving for action upon the adjustable-length tappet (Fig. 3: 80) first releases energy which was stored in it by axial compression in order to produce an axial force for insertion of the needle (74) of the injection device, and then releases energy which was stored in this spring by torsion in order to produce a torque for injection of the quantity of fluid to be injected.

5. Injection device according to one or more of claims 2 to 4, characterised in that the blocking device (42, 118) is not operative in the proximal end position (Fig. 10) of the tappet (80).

6. Injection device according to one or more of claims 2 to 4, and claim 5, characterised in that in the distal end position (Fig. 10) of the tappet (80) the adjusting member (15) is adjustable by rotation beginning at a predetermined rotary position (stop 44), and in that the rotation of the adjusting member (15) in the proximal end position (Fig. 7), which proceeds in the opposite direction in that position, is limited by reaching this predetermined rotary position (stop 44).

7. Injection device according to one or more of claims 2 to 6, characterised in that the blocking device comprises a pawl (118) disposed on the housing (100) and ratchet teeth (42) connected to the adjusting member (15), or vice versa, said pawl (118) engaging in said ratchet teeth (42) in the distal end position (Fig. 10) of the tappet (80).

8. Injection device according to one or more of the preceding claims, characterised in that the guide member (67) is rotatably but axially non-displaceably coupled to the adjusting member (15).

9. Injection device according to claim 8, characterised in that at least one elastically deformable element (63, 64) is provided between the guide member (67) and the adjusting member (15), said deformable element (63, 64) being disposed with biasing between the guide member (67) and the adjusting member (15) and being positively coupled to at least one of these two members (15, 67).

10. Injection device according to claim 8 or 9, characterised in that the guide member (67) is provided with at least one engagement element (72) which in the proximal end position (Fig. 7) of the tappet (Fig. 3: 80) is in engagement with at least one engagement element (93) of the injection device (10) which is substantially complementary with it and is structurally connected to the housing.

11. Injection device according to claim 10, characterised in that the engagement element of the guide member (67) is a set of lengthwise teeth (72), and in that a corresponding set of lengthwise teeth (93) structurally connected to the housing is associated with this set of lengthwise teeth (72) and is in engagement with the set of lengthwise teeth (72) of the guide member (67) at least in the proximal end position (Fig. 7) of the tappet (Fig. 3: 80).

12. Injection device according to claims 7 and 11, characterised in that the ratchet teeth (42) and the set of lengthwise teeth (72) have a tooth pitch (Figs. 11 and 14: angle alpha) which is compatible with one another.

13. Injection device according to claim 12, characterised in that the tooth pitch of the ratchet teeth (42) and the tooth pitch of the set of lengthwise teeth (72) have values such that the larger value can be divided integrally by the smaller value.

14. Injection device according to claim 12 or 13, characterised in that the teeth of the set of lengthwise teeth (72) are disposed in such a way relative to the pawl (118) associated with the ratchet teeth (42) that upon a movement of the tappet (Fig. 3: 80) from its distal end position (Fig. 10) to its proximal end position (Fig. 7) a substantially rotation-free linear motion of the tappet (80) and of the guide member (67) disposed on it is enabled.

15. Injection device according to one or more of the preceding claims, characterised in that the threaded spindle (19) is made of plastic and has lengthwise grooves (29, 30) disposed symmetrically with respect to one another for engagement with a guide element (69) of the guide member (67).

16. Injection device according to one or more of the preceding claims, characterised in that the threaded spindle (19) is sub-divided longitudinally into two visually distinguishable regions (24, 25).

17. Injection device according to claim 16, characterised in that one region (25) of the threaded spindle (19) is coloured.

18. Injection device according to one or more of the preceding claims, characterised in that the guide member (67) is adapted to displace the cartridge (11), or a cartridge holder (148, 162) receiving it, in the proximal direction (Fig. 19: arrows 164) during the injection process by direct contact with the cartridge or cartridge holder.

19. Injection device according to one or more of claims 2 to 18, characterised in that upon the motion of the tappet (Fig. 3: 80) from its distal end position (Fig. 10) to its proximal end position (Fig. 7), the blocking device (42, 118) is adapted to block the rotation of the adjusting member (15) in said opposite direction of rotation at least as long as the guide member (67) is freely rotatable relative to the housing (100).

20. Injection device according to one or more of the preceding claims, characterised in that the adjusting member (15) is coupled to an actuation device (38) located outside the housing (100), said actuation device enabling the adjustment of the tappet (Fig. 3: 80) from its proximal end position (Fig. 7) to its distal end position (Fig. 10) while simultaneously cocking the spring (53) associated with the tappet (80), said actuation device also enabling, in the distal end position (Fig. 10), a rotation of the actuation device (38) for pre-selecting a desired injection dose (Fig. 6: 46).

21. Injection device according to claim 20, characterised in that a releasable detent device (59, 104) is associated with the tappet (Fig. 3: 80) for locking into place in the distal end position (Fig. 10) of the tappet.

22. Injection device according to one or more of the preceding claims, characterised in that a scale (Fig. 6: 46) for reading the set dose is associated with the actuation device (38), said scale being reset to zero upon the motion occurring on expelling the preset injection dose.

23. Injection device for holding a cartridge (11) containing a quantity of injection fluid (12) typically adequate for multiple injections, said cartridge (11) being displaceable in a proximal direction in the injection device (10) counter to the force of a resetting spring (157), comprising an adjustable-length tappet (Fig. 3: 80) acted upon in the proximal direction by a spring (53), said tappet being displaceable in the injection device (10) between a proximal end position (Fig. 7) and a distal end position (Fig. 10) and comprising a threaded spindle (19), guided in the thread (17) of an adjusting member (15) for action upon a plunger (23) provided in the cartridge (11), a guide member (67) being associated with said threaded spindle, and coupled thereto in a manner fixed against relative rotation but axially displaceably, characterised in that the guide member (67) is adapted to act, during the proximal displacement of the cartridge (11) taking place in the injection process, upon this cartridge (11), or a cartridge holder (148, 162) receiving it, directly (Fig. 19: arrows 164) in order to displace it, and in that the guide member (67) is rotatable relative to the housing (100) of the injection device (10) in the distal end position (Fig. 10) of the tappet (80), but not in the proximal end position (Fig. 7).

24. Injection device according to claim 23, characterised in that a spring (53) is provided between the housing (100) and the adjusting member (15), whose biasing is variable by rotation of the adjusting member (15), and in that a blocking device (42, 118) is provided in the distal end position (Fig. 10) of the tappet (80), said blocking device enabling such rotation of the adjusting member (15) only in a predetermined direction and blocking it in the opposite direction.

25. Injection device according to claim 23 and 24, characterised in that the spring (53), whose biasing is variable by rotation of the adjusting member (15), also serves for action upon the adjustable-length tappet (Fig. 3: 80) in the proximal direction.

26. Injection device according to claim 25, characterised in that during the injection process the spring (53) serving for action upon the adjustable-length tappet (Fig. 3: 80) first releases energy which was stored in it by axial compression in order to produce an axial force for insertion of the needle (74) of the injection device, and then releases energy which was stored in this spring by torsion in order to produce a torque for injection of the quantity of fluid to be injected.

27. Injection device according to one or more of the preceding claims, characterised in that the blocking device (42, 118) is not operative in the proximal end position (Fig. 7) of the tappet (80).

## Revendications

1. Injecteur destiné à recevoir une cartouche (11) avec une quantité de liquide d'injection (12) suffisant habituellement pour une pluralité d'injections,
ladite cartouche (11) pouvant être déplacée en direction proximale dans l'injecteur (10) à l'encontre de la force d'un ressort de rappel (157), comprenant un poinçon (figure 3 : 80) sollicité en direction proximale par un ressort (53) et réglable en longueur, ledit poinçon pouvant être déplacé dans l'injecteur (10) entre une position finale proximale (figure 7) et une position finale distale (figure 10) et comportant une tige filetée (19) qui est guidée dans un taraudage (17) d'un organe de réglage (15), ladite tige filetée servant à solliciter un piston (23) prévu dans la cartouche (11) et étant associée à un organe de guidage (67) qui est relié à la tige filetée solidairement en rotation mais avec faculté de se déplacer axialement,
caractérisé en ce que l'organe de guidage (67) est capable de tourner par rapport au boîtier (100) de l'injecteur (10) dans la position finale distale (figure 10) du poinçon (80), mais non pas dans la position finale proximale (figure 7).

2. Injecteur selon la revendication 1, caractérisé en ce qu'il est prévu entre le boîtier (100) et l'organe de réglage (15) un ressort (53) dont la précontrainte peut être modifiée en faisant tourner l'organe de réglage (15), et en ce que dans la position finale distale (figure 10) du poinçon (80) il est prévu un dispositif de blocage (42, 118) qui permet une telle rotation de l'organe de réglage (15) uniquement dans une direction déterminée, et bloque une rotation dans la direction opposée.

3. Injecteur selon les revendications 1 et 2, caractérisé en ce que le ressort (53), dont la précontrainte peut être modifiée en faisant tourner l'organe de réglage (15) sert également à solliciter le poinçon réglable en longueur (figure 3 : 80) en direction proximale.

4. Injecteur selon la revendication 3, caractérisé en ce que le ressort (53) qui sert à solliciter le poinçon réglable en longueur (figure 3 : 80) libère lors de l'opération d'injection d'abord de l'énergie qui a été stockée dans ce ressort par compression axiale, afin de produire une force axiale pour enfoncer l'aiguille (74) de l'injecteur, et libère ensuite de l'énergie qui a été stockée dans ce ressort par torsion, afin de produire un couple de rotation pour l'injection de la quantité de liquide à injecter.

5. Injecteur selon l'une ou plusieurs des revendications 2 à 4, caractérisé en ce que le dispositif de blocage (42, 118) n'est pas actif dans la position finale proximale (figure 10) du poinçon (80).

6. Injecteur selon l'une ou plusieurs des revendications 2 à 4 et selon la revendication 5, caractérisé en ce que l'organe de réglage (15), dans la position finale distale (figure 10) du poinçon (80), peut être déplacé par rotation à partir d'une position de rotation prédéterminée (butée 44), et en ce que la rotation de l'organe de réglage (15) dans la position finale proximale (figure 7), qui s'y déroule en direction opposée, est limitée jusqu'à cette position de rotation prédéterminée (butée 44).

7. Injecteur selon l'une ou plusieurs des revendications 2 à 6, caractérisé en ce que le dispositif de blocage comporte un cliquet d'arrêt (118) agencé sur le boîtier (100) et des dents de cliquet (42) reliées à l'organe de réglage (15), ou inversement, le cliquet d'arrêt (118) s'engageant dans la position finale distale (figure 10) du poinçon (80) dans ces dents de cliquet (42).

8. Injecteur selon l'une ou plusieurs des revendications précédentes, caractérisé en ce que l'organe de guidage (67) est relié à l'organe de réglage (15) de façon à pouvoir tourner, mais sans pouvoir se déplacer axialement.

9. Injecteur selon la revendication 8, caractérisé en ce qu'il est prévu entre l'organe de guidage (67) et l'organe de réglage (15) au moins un élément élastiquement déformable (63, 64), qui est agencé sous précontrainte entre l'organe de guidage (67) et l'organe de réglage (15), et est relié en coopération de formes à l'un au moins de ces deux organes (15, 67).

10. Injecteur selon l'une ou l'autre des revendications 8 ou 9, caractérisé en ce que l'organe de guidage (67) est pourvu d'au moins un élément d'engagement (72), lequel est en engagement, dans la position finale proximale (figure 7) du poinçon (figure 3 : 80), avec au moins un élément d'engagement (93) de l'injecteur (10), solidaire du boîtier et sensiblement complémentaire du poinçon.

11. Injecteur selon la revendication 10, caractérisé en ce que l'élément d'engagement de l'organe de guidage (67) est réalisé sous la forme d'une denture longitudinale (72), et en ce qu'à cette denture longitudinale (72) est associée une denture longitudinale correspondante (93) solidaire du boîtier, laquelle est en engagement avec la denture longitudinale (72) de l'organe de guidage (67) au moins dans la position finale proximale (figure 7) du poinçon (figure 3 : 80).

12. Injecteur selon les revendications 7 et 11, caractérisé en ce que les dents de cliquet (42) et la denture longitudinale (72) présentent des pas de denture (figures 11 et 14 ; angle α) compatibles l'un avec l'autre.

13. Injecteur selon la revendication 12, caractérisé en ce que le pas des dents de cliquet (42) et le pas de la denture longitudinale (72) ont des valeurs telles que la plus élevée est divisible par la plus faible d'un nombre entier.

14. Injecteur selon l'une ou l'autre des revendications 12 et 13, caractérisé en ce que les dents de la denture longitudinale (72) sont agencées de telle manière, par rapport au cliquet d'arrêt (118) associé aux dents de cliquet (42) que, lors d'un déplacement du poinçon (figure 3 : 80) depuis sa position finale distale (figure 10) jusqu'à sa position finale proximale (figure 7), un déplacement linéaire du poinçon (80) sensiblement exempt de rotation, et de l'organe de guidage (67) agencé sur lui-même, est possible.

15. Injecteur selon l'une ou plusieurs des revendications précédentes, caractérisé en ce que la tige filetée (19) est réalisée en matière plastique, et présente des gorges longitudinales agencées symétriquement l'une par rapport à l'autre (29, 30), pour engagement avec un élément de guidage (69) de l'organe de guidage (67).

16. Injecteur selon l'une ou plusieurs des revendications précédentes, caractérisé en ce que la tige filetée (19) est subdivisée en direction longitudinale en deux régions (24, 25) capables d'être distinguées visuellement.

17. Injecteur selon la revendication 16, caractérisé en ce qu'une région (25) de la tige filetée (29) est réalisée en couleur.

18. Injecteur selon l'une ou plusieurs des revendications précédentes, caractérisé en ce que l'organe de guidage (67) est réalisé de manière à déplacer, lors de l'opération d'injection, la cartouche (11) ou un porte-cartouche (148, 162) qui reçoit celle-ci, en direction proximale (figure 19 : flèches 164) par contact direct contre cette cartouche ou ce porte-cartouche.

19. Injecteur selon l'une ou plusieurs des revendications 2 à 18, caractérisé en ce que le dispositif de blocage (42, 118) est réalisé de telle manière qu'il bloque la rotation de l'organe de réglage (15) dans la direction de rotation opposée précitée, lors du déplacement du poinçon (figure 3 : 80) depuis sa position finale distale (figure 10) jusqu'à sa position finale proximale (figure 7) au moins aussi longtemps que l'organe de guidage (67) est capable de tourner librement par rapport au boîtier (100).

20. Injecteur selon l'une ou plusieurs des revendications précédentes, caractérisé en ce que l'organe de réglage (15) est relié à un organe d'actionnement (38) agencé à l'extérieur du boîtier (100), ledit organe permettant le déplacement du poinçon (figure 3 : 80) depuis sa position finale proximale (figure 7) jusqu'à sa position finale distale (figure 10), en bandant simultanément le ressort (53) associé au poinçon (80), et permettant dans la position finale distale (figure 10) une rotation de l'organe d'actionnement (38) pour la présélection d'une dose d'injection désirée (figure 6 : 46).

21. Injecteur selon la revendication 20, caractérisé en ce qu'un organe d'enclenchement libérable (59, 104) est associé au poinçon (figure 3 : 80) pour son enclenchement dans sa position finale distale (figure 10).

22. Injecteur selon l'une ou plusieurs des revendications précédentes, caractérisé en ce qu'une échelle (figure 6 : 46) pour la lecture de la dose réglée est associée à l'organe de positionnement (38), ladite échelle étant ramenée à zéro lors du mouvement qui se produit en éjectant la dose d'injection auparavant réglée.

23. Injecteur destiné à recevoir une cartouche (11) avec une quantité de liquide d'injection (12) suffisant habituellement pour une pluralité d'injections, ladite cartouche (11) pouvant être déplacée dans l'injecteur (10) en direction proximale à l'encontre de la force d'un ressort de rappel (157),
comprenant un poinçon (figure 3 : 80) réglable en longueur et sollicité par un ressort (53) en direction proximale, ledit poinçon pouvant être déplacé dans l'injecteur (10) entre une position finale proximale (figure 7) et une position finale distale (figure 10) et comportant une tige filetée (19) qui est guidée dans un taraudage (17) d'un organe de réglage (15), ladite tige filetée servant à solliciter un piston (23) prévu dans la cartouche (11) et étant associée à un organe de guidage (67) qui lui est relié solidairement en rotation mais avec faculté de déplacement axial,
caractérisé en ce que l'organe de guidage (67) est réalisé, lors du déplacement proximal de la cartouche (11) qui se produit pendant l'opération d'injection, de manière à solliciter (figure 19 : flèches 164) directement ladite cartouche (11), ou un porte-cartouche (148, 162) qui la reçoit, et à la déplacer,
et en ce que l'organe de guidage (67) peut être tourné par rapport au boîtier (100) de l'injecteur (10) dans la position finale distale (figure 10) du poinçon (80), mais non pas dans la position finale proximale (figure 7).

24. Injecteur selon la revendication 23, caractérisé en ce qu'il est prévu, entre le boîtier (100) et l'organe de réglage (15), un ressort (53) dont la précontrainte peut être modifiée par rotation de l'organe de réglage (15), et en ce que, dans la position finale distale (figure 10) du poinçon (80), est prévu un dispositif de blocage (42, 118) qui permet une telle rotation de l'organe de réglage (15) uniquement dans une direction déterminée, et bloque une rotation dans la direction opposée.

25. Injecteur selon les revendications 23 et 24, caractérisé en ce que le ressort (53), dont la précontrainte peut être modifiée par rotation de l'organe de réglage (15), sert également à solliciter, en direction proximale, le poinçon (figure 3 : 80) réglable en longueur.

26. Injecteur selon la revendication 25, caractérisé en ce que le ressort (53) qui sert à solliciter le poinçon (figure 3 : 80) réglable en longueur libère, lors de l'opération de l'injection, tout d'abord de l'énergie qui a été stockée en lui-même par compression axiale, afin de produire une force axiale pour enfoncer l'aiguille (74) de l'injecteur,
et libère ensuite de l'énergie qui a été stockée dans ce ressort par torsion, afin de produire un couple de rotation pour l'injection de la quantité de liquide à injecter.

27. Injecteur selon l'une ou plusieurs des revendications précédentes, caractérisé en ce que le dispositif de blocage (42, 118) n'est pas actif dans la position finale proximale (figure 7) du poinçon (80).
